# EUROPÄISCHE PATENTSCHRIFT

(11) **EP 3 180 033 B1**
(45) Veröffentlichungstag und Bekanntmachung des Hinweises auf die Patenterteilung: **07.06.2023**
(21) Anmeldenummer: 15759653.7
(22) Anmeldetag: 13.08.2015
(51) Int. Cl.: A61K 47/50, A61K 47/64, A61K 48/00

(54) **PEPTID ZUR VERWENDUNG IN DER REDUKTION VON NEBENWIRKUNGEN IN FORM VON IMMUNSTIMULATORISCHEN REAKTIONEN/EFFEKTEN**
PEPTIDE FOR USE IN THE REDUCTION OF SIDE EFFECTS IN THE FORM OF IMMUNOSTIMULATORY REACTIONS/EFFECTS
PEPTIDE DESTINÉE À ÊTRE UTILISÉE DANS LA RÉDUCTION D'EFFETS SECONDAIRES SOUS FORME DE RÉACTIONS/EFFETS IMMUNOSTIMULATEURS

(30) Priorität: 14.08.2014 EP 14181072
(43) Veröffentlichungstag der Anmeldung: 21.06.2017
(73) Patentinhaber: Friedrich-Schiller-Universität Jena, 07743 Jena (DE)
(72) Erfinder: PÖHLMANN, Tobias, 08058 Zwickau (DE); GÜNTHER, Rolf, 22559 Hamburg (DE); REUTER, Michael, 07749 Jena (DE); LUDWIG, Mirko, 07743 Jena (DE)
(74) Vertreter: Vossius & Partner Patentanwälte Rechtsanwälte mbB
(86) Internationale Anmeldenummer: PCT/EP2015/068634
(87) Internationale Veröffentlichungsnummer: WO 2016/023974

(56) Entgegenhaltungen:
- EP-A1- 2 216 047
- WO-A1-2014/043544
- WO-A1-2014/076213
- WO-A2-2008/098569
- SHINICHIRO INABA ET AL: "Atelocollagen-mediated Systemic Delivery Prevents Immunostimulatory Adverse Effects of siRNA in Mammals", MOLECULAR THERAPY, Bd. 20, Nr. 2, 25. Oktober 2011 (2011-10-25), Seiten 356-366, XP055167631, ISSN: 1525-0016, DOI: 10.1038/mt.2011.221
- MINAKUCHI YOSHIKO ET AL: "Atelocollagen-mediated synthetic small interfering RNA delivery for effective gene silencing in vitro and in vivo", NUCLEIC ACIDS RESEARCH, OXFORD UNIVERSITY PRESS, GB, Bd. 32, Nr. 13, 1. Januar 2004 (2004-01-01), Seiten E109-1, XP002567698, ISSN: 0305-1048, DOI: 10.1093/NAR/GNH093 [gefunden am 2004-07-22]
- S. E. EWALD ET AL: "Nucleic acid recognition by Toll-like receptors is coupled to stepwise processing by cathepsins and asparagine endopeptidase", NATURE IMMUNOLOGY, Bd. 7, Nr. 2, 14. März 2011 (2011-03-14), Seiten 156-651, XP055167213, ISSN: 1529-2908, DOI: 10.1038/ni1297
- A. GARCIA-CATTANEO ET AL: "Cleavage of Toll-like receptor 3 by cathepsins B and H is essential for signaling", PROCEEDINGS OF THE NATIONAL ACADEMY OF SCIENCES, Bd. 109, Nr. 23, 18. Mai 2012 (2012-05-18) , Seiten 9053-9058, XP055167216, ISSN: 0027-8424, DOI: 10.1073/pnas.1115091109

## Beschreibung

Die Erfindung betrifft ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül gekoppelt ist. Außerdem betrifft die Erfindung ein solches Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül gekoppelt ist, durch das die immunstimulatorischen Reaktionen/Effekte ausgelöst werden und sich beispielsweise in der Aktivierung/Expression von IFIT1/2, IRF9, TLR3, TLR7, TLR8 oder PKR auszeichnen. Somit betrifft die Erfindung insbesondere ein solches Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül gekoppelt ist, wobei die immunstimulatorischen Reaktionen/Effekte ausgelöst werden durch eine Aktivierung von IFIT1/2, IRF9, TLR3, TLR7, TLR8 oder PKR.

Bekannt ist, Zellen gezielt durch die Einbringung von Nukleinsäuren zu beeinflussen. Beispielsweise kann durch siRNA oder Antisense-Nukleinsäuren die Expression von Genen vermindert werden. Durch saRNAs kann die Expression von Genen verstärkt werden, durch die Einbringung von mRNAs können Proteine in Zellen hergestellt werden. Es ist außerdem beschrieben, dass Nukleinsäuren zum Zweck der Beeinflussung von Zellen beispielsweise einzelsträngig, doppelsträngig, kurz (10-30 Basen) oder langkettig (30-10000 Basen) verwendet werden können. Zur Erhöhung der Stabilität und zum Schutz vor Nukleasen können die Nukleinsäuren chemisch modifiziert werden. Zur Einbringung in Zellen und zur Selektivität für bestimmte Zellen können sogenannte Delivery-Reagenzien (beispilsweise Lipide, Polyethylenimine oder Nanopartikel), Zellpenetrierende Peptide, Proteasesubstrate, Antikörper, Antikörperfragmente oder weitere Strukturen an die Nukleinsäuremoleküle gebunden werden.

Im Stand der Technik ist bereits beschrieben, dass ein systemisches Delivery, das durch Atelocollagen vermittelt wird, immunstimulatorische Nebenwirkungen durch siRNA verhindert wird (Inaba et al., Molecular Therapy 20(2):356-366 (2011)).

Zudem sind in WO 2008/098569 siRNA-Konstrukte beschrieben, die an für bestimmte Proteasen spezifische Peptide gekoppelt sind. Es ist beschrieben, dass diese Peptide zellspezifisch abgespalten werden können, wodurch die siRNA-Moleküle eine definierte Zellspezifität entfalten können.

Allerdings ist es bekannt, dass vor Allem *in vivo* bei der Verwendung von Nukleinsäuren häufig das Problem auftritt, dass in Zellen immunregulatorische Effekte ausgelöst werden und eine Toll-like-Rezeptor-(TLR)-Aktivierung stattfindet. In Folge dessen kann es zu einer Expression von Interferonen kommen. Hierdurch werden Folgeprozesse in Zellen ausgelöst. Beispielsweise kann es zu eine generellen Expressionshemmung und einer Zytotoxizität kommen. *In vivo* können diese zellulären Prozesse erhebliche Folgen haben, beispielsweise Rötungen, Entzündungen, grippeähnliche Symptome und Fieber.

Außerdem besteht *in vivo* die besondere Gefahr, dass beispielsweise durch liposomale Delivery-Strategien die Nukleinsäuren an der Zelloberfläche haften bleiben und dadurch neben zytoplasmatischen oder endosomalen auch extrazelluläre bzw. in der Zellmembran vorhandene TLR-Rezeptoren aktiviert werden und dadurch verstärkt systemische Effekte und Immunreaktionen auftreten.

Diese Nebeneffekte bzw. Nebenwirkungen stellen ein großes Problem im Rahmen von Gentherapien dar, bei denen man eigentlich durch Einbringen von Nukleinsäuren wie DNA oder (si)RNA in die Körperzellen eines Individuums eine gezielte Behandlung bestimmter Krankheiten erreichen will. So kann beispielsweise ein DNA-Molekül bzw. ein bestimmtes Gen eingebracht werden, das ein Protein codiert und das in den Zellen (über)exprimiert werden kann, um dadurch beispielsweise einen Gendefekt im endogenen Gen der Zelle zu kompensieren. Durch siRNA-Moleküle kann beispielsweise gezielt die Expression eines Gens über den bekannten Mechanismus der RNA Interferenz vermindert oder vollständig eliminiert werden, um dadurch beispielsweise eine Erkrankung, für die ursächlich eine (krankheitsauslösende) Expression eines bestimmten Gens verantwortlich ist, zu therapieren.

Diese unerwünschten Nebenwirkungen bei Gentherapien können derzeit im Stand der Technik, zumindest teilweise vermindert werden, indem das Zucker-Phosphat-Rückgrad beispielsweise der siRNA chemisch modifiziert wird, beispielsweise durch MeO-Modifikation. Allerdings bieten die genannten Modifikationen keinen ausreichenden Schutz vor der Induktion unspezifischer Effekte.

Deshalb besteht ein Bedarf daran, Mittel und Wege zu finden, um Nebenwirkungen in Form der beschriebenen, unspezifischen Effekte, die bei der Verwendung von Nukleinsäuren auftreten, zu minimieren oder zu umgehen.

Der Erfindung liegt deshalb die Aufgabe zu Grunde, Modifikationen der Nukleinsäure-Sequenzen bereitzustellen, die die genannten Nebenwirkungen und unspezifischen Effekte minimieren oder umgehen.

Die Erfindung beruht auf dem überraschenden Befund, dass sich durch die Kopplung eines Peptids an ein in der Gentherapie eingesetztes Nukleinsäuremolekül die als unerwünschte Nebenwirkung auftretenden immunstimulatorischen Reaktionen/Effekte drastisch reduzieren lassen. Somit betrifft die Erfindung ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist.

Die vorliegende Erfindung betrifft die in den Ansprüchen beschriebenen Gegenstände. Somit betrifft die vorliegende Erfindung die folgenden Punkte.
1. Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül gekoppelt ist, wobei das Peptid aus bis zu 300 Aminosäuren besteht, wobei das Peptid die Spaltsequenz für Legumain (Spaltsequenz: Ala-Ala-Asn) umfasst.
2. Peptid zur Verwendung nach Punkt 1, wobei die immunstimulatorischen Reaktionen/Effekte ausgelöst werden durch eine Aktivierung von IFIT1/2, IRF9, TLR3, TLR7, TLR8 oder PKR.
3. Peptid zur Verwendung nach Punkt 1, wobei die immunstimulatorischen Reaktionen/Effekte zu Rötungen, Entzündungen, grippeähnlichen Symptomen und/oder Fieber führen.
4. Peptid gekoppelt an ein Nukleinsäuremolekül nach einen der Punkte 1 bis 3, wobei das Nukleinsäuremolekül weitere chemische Modifikationen umfasst.
5. Peptid zur Verwendung nach einem der Punkte 1 bis 4, wobei die Spaltsequenz eine Sequenz mit Deletion, Substitution, Insertion und/oder Hinzufügung von 1 bis 10 Aminosäuren in der Sequenz Ala-Ala-Asn umfasst, und die durch Legumain gespaltet werden kann.
6. Peptid zur Verwendung nach einen der Punkte 1 bis 5, wobei das Nukleinsäuremolekül ausgewählt ist aus der Gruppe bestehend aus mRNA, siRNA, miRNA, saRNA, RNA, PNA, DNA, LNA, Gemischen aus verschiedenen Nukleotiden und Aptameren.
7. Peptid zur Verwendung nach einen der Punkte 1 bis 6, wobei das Nukleinsäuremolekül eine siRNA ist.
8. Peptid zur Verwendung nach Punkt 7, wobei die siRNA eine Nucleotidsequenz umfasst, die ausgewählt ist aus der Gruppe bestehend aus:
   (5-3) UCA UAU UCG ACU UUG GUU GCC (Polo-like Kinase (PLK); SEQ ID NO:8);
   (5-3) UCA AAC UCC AUC AUG AUC U (SEQ ID NO:9) und/oder (5-3) UCC AUC AUG AUC UUC UGG A (SEQ ID NO:10) (CHMP);
   (5-3) UUC AUA AAC ACA GUU CUC C (SEQ ID NO:11) (PDCD);
   (5-3) UCA AAU UGA GGC ACU GUG C (SEQ ID NO:12) (RFWD);
   (5-3) UUU CUU CAG AGC AGG AGC A (SEQ ID NO:13), (5-3) AUA CAC ACC CUU UGC CUC A (SEQ ID NO:14) und/oder (5-3) AUU UCA GGC UCA UAU UCC U (SEQ ID NO:15) (ATAP);
   (5-3) CAC AAU UCC CAC UUU GAG C (SEQ ID NO:16), (5-3) GUU ACC CAC AAU UCC CAC U (SEQ ID NO:17) und/oder (5-3) UUU CUU CUC UUU GUC UGG G (SEQ ID NO:18) (AGAP); und
   (5-3) UAU UCU CCA AAC AAU GUG C (SEQ ID NO:19) (RCHY).
9. Peptid zur Verwendung nach einem der Punkte 1 bis 8, wobei das Peptid/Nukleinsäurekonstrukt zur Einbringung in Zellen und/oder zum gezielten Einbringen in spezifische Zellen an Moleküle, wie Zell-penetrierende Peptide und/oder Enzymsubstrate, und/oder an Reagenzien, wie Polyethylenimine, Nanocontainer, Nanopartikel oder Lipide, und/oder an Rezeptor-Liganden-Komplexe kovalent oder nicht-kovalent gebunden sind.

Wie in den beigefügten Beispielen beschrieben, wurde bei der Analyse der immunstimulatorischen Effekte von Nukleinsäuren am Beispiel von siRNA und siRNA-Modifikationen überraschend gefunden, dass die Expression von Genen, welche mit einer TLR-Reaktion auf siRNA in Verbindung gebracht werden, dadurch vermieden werden kann, dass die siRNA am Antisense-Strang an ein Peptid gebunden wird. Dies ist insbesondere deshalb unerwartet, da im Stand der Technik nicht beschrieben ist, dass die Kopplung von Peptiden einen Einfluss auf unspezifischen Effekte und Nebenwirkungen in Form von immunstimulatorischen Reaktionen/Effekten hat, die sonst bei der Verwendung von Nukleinsäuren im Rahmen von Gentherapien auftreten. Insbesondere ist es überraschend, dass diese unspezifischen Effekte und Nebenwirkungen in Form von immunstimulatorischen Reaktionen/Effekten drastisch durch die Bindung eines Peptids reduziert werden können. Dies ist auch deshalb von Bedeutung, da es immer ein Bestreben ist, bei der Behandlung von Krankheiten auftretende Nebenwirkungen zu minimieren oder zu eliminieren und die vorliegende Erfindung einen einfachen Weg bereitstellt, um Nebenwirkungen in Form der beschriebenen unspezifischen Effekte, die bei der Verwendung von Nukleinsäuremolekülen auftreten, zu minimieren oder zu umgehen, denn eine einfache Peptid-Kopplung der in der Gentherapie eingesetzten Nukleinsäuremolekülen ist leicht umzusetzen.

Wie in den beigefügten Beispielen gezeigt, ist die Expression von IFIT1 (Interferon induced protein with tetratricopeptid repeats 1; ein Marker für die Induktion unspezifischer immunstimulatorischer Effekte) durch die Einbringung von 4 verschiedenen siRNA Sequenzen (Zielgene AGAP, ATAP, RFWD, CHMP) in MCF7-Zellen stark erhöht. Die Beispiele zeigen, dass durch die kovalente Bindung eines Peptides (im vorliegenden Beispiel am 5'Phosphat-Ende des Antisense-Stranges über einen Amino-C6-Linker) eine im Vergleich zu der nicht-Peptid-gebundenen siRNA eine stark verminderte IFITl-Expression detektiert wird, wobei in einigen Fällen die IFIT1-Expression vergleichbar mit der unbehandelter Zellen war. Somit konnte überraschend gezeigt werden, dass unabhängig von der verwendeten Nukleinsäure und der entsprechenden Zielsequenz, durch die Kopplung eines Peptids immunstimulatorische Reaktionen/Effekte reduziert werden können. Außerdem ist die Reduktion dieser immunstimulatorischen Reaktionen auch unabhängig vom verwendeten Peptid, da gezeigt werden konnte, dass die IFIT1 Expression mit unterschiedlichen Peptiden gleichermaßen reduziert werden konnte.

Die vorliegende Erfindung stellt also ein Peptid bereit, zur Verwendung in der Reduktion von Nebenwirkungen in Form von (unspezifischen) immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist.

Das in der Gentherapie eingesetzte Nukleinsäuremolekül ist nicht auf ein bestimmtes Molekül oder gar auf eine spezifische Nukleinsäuresequenz beschränkt. Vielmehr kann jedes Nukleinsäuremolekül verwendet werden, das im Rahmen einer Gentherapie eingesetzt werden kann oder eingesetzt werden soll. Die Wahl des Nukleinsäuremoleküls, das in der Gentherapie eingesetzt werden soll, hängt sehr stark von der zu behandelten Krankheit ab, die im Rahmen der Gentherapie behandelt werden soll. Beispiele werden weiter unten genannt.

Als Gentherapie bezeichnet man allgemein in der Medizin das Einfügen oder Einbringen von Genen oder Nukleinsäuremolekülen in Zellen oder Gewebe eines Menschen, um Erbkrankheiten, Gendefekte oder Stoffwechselerkrankungen, Tumorerkrankungen und Virus-Infektionen zu behandeln. Für den ungezielten Transfer, also dem Einbringen der Gene oder Nukleinsäuremoleküle, sind im Stand der Technik verschiedene Methoden bekannt, die in der Regel sogenannte Vektoren verwenden, um ein therapeutisches Gen/Nukleinsäuremolekül in eine Zelle zu transportieren. Beispielsweise können die Gene/Nukleinsäuremoleküle durch (chemische) Transfektion in die Zellen gebracht werden. Dabei werden die therapeutischen Gene und eine elektrisch geladene Verbindung (z.B. Calciumphosphat) zu den Zellen gegeben. Die elektrisch geladene Verbindung stört die Struktur der Zellmembran, wodurch das zu übertragende Nukleinsäuremolekül ins Zellinnere gelangen kann. Durch (physikalische) Transfektion kann das Nukleinsäuremolekül z.B. durch Elektroporation, mit einer Particle gun oder durch Erythrozyten-Ghosts in die Zelle transferiert werden. Im Rahmen einer klinischen Anwendung wird das zu übertragene Nukleinsäuremolekül allerdings bevorzugt mittels Transduktion in die Zelle übertragen. Dabei bringt ein Virus das therapeutische Gen in die Zelle. Bei der Transduktion gibt es mehrere mögliche Transportviren, die dem Fachmann bekannt sind. Am häufigsten werden sogenannte retrovirale Systeme verwendet.

Außerdem werden therapeutische Gene/Nukleinsäuremoleküle, insbesondere bei synthetisch hergestellten Peptid-siRNA-Konstrukten, mittels Lipiden, Lipid-Nanopartikeln oder Polymeren in Zellen oder Gewebe eingebracht. Diese Verfahren sind im Stand der Technik bekannt. Außerdem können diese Konstrukte mit oder ohne "core-shell-Aufbau" versehen sein. Unter "core-shell-Aufbau" versteht man eine Möglichkeit der "Verpackung" eines Wirkstoffs, bei der beispielsweise ein negativ geladenes Molekül (wie z.B. ein Peptid/Nukleinsäuremolekül der vorliegenden Erfindung, bevorzugt eine siRNA gekoppelt an ein Peptid) in eine Hülle aus positiv geladenen Molekülen eingehüllt wird, die dann wiederum beispielsweise durch eine weitere Hülle aus Polyethylenglycol (PEG) verpackt wird. PEG wird häufig eingesetzt, um die Plasmahalbwertszeit zu erhöhen, da PEG in der Lage ist, die Nierendurchgängigkeit zu verringern. Diese Konstrukte können zudem mit einer weiteren Schicht umgeben sein, die die Zellgängigkeit, den Eintritt in die Zelle, gewährleisten. Entsprechende Möglichkeiten, die erfindungsgemäßen Peptid/Nukleinsäuremolekül-Konstrukte mit einem "core-shell-Aufbau" zu versehen, sind im Stand der Technik beschrieben. In diesem Zusammenhang sei beispielsweise auf den Übersichtsartikel von Tam et al., Pharmaceutics 5(3): 498-507 (2013) verwiesen. Zudem können die Konstrukte in PEG eingebunden sein, was die Funktion eines "stealth-Mechanismus" erfüllt. Unter "stealth-Mechanismus" versteht man die Möglichkeit, die bei der "Verpackung" von Wirkstoffen genutzt wird, Moleküle wie PEG in die äußere Hülle der "Verpackung" einzubringen, die in der Lage sind, vor Reaktionen des Immunsystems gegen weitere, in der Hülle enthaltene Moleküle/Proteine zu schützen. Ferner können die Konstrukte über im Stand der Technik bekannte Targeting-Moleküle (wie beispielsweise spezifische Antikörper, deren Fragmente oder Peptide) verfügen, die gezielt bestimmte zu behandelnde Zellen erkennen können. Dem Fachmann sind beispielsweise eine Vielzahl verschiedener Zelloberflächenmoleküle, Antigene oder Epitope bekannt, die spezifisch für bestimmte Zellen oder Gruppen von Zellen sind. So können entsprechende spezifische Antikörper, deren Fragmente oder Peptide, die diese Zelloberflächenmoleküle, Antigene oder Epitope erkennen und binden können, genutzt werden, damit die erfindungsgemäßen Peptid/Nukleinsäuremolekül-Konstrukte, die in der Gentherapie eingesetzt werden, gezielt bestimmte Zellen, in die die Konstrukte eingebracht werden sollen, finden und erkennen (und somit eingebracht werden) können.

Wie bereits oben erwähnt, treten häufig bei in der Gentherapie eingesetzten Nukleinsäuremoleküle Nebenwirkungen in Form von (unspezifischen) immunregulatorischen bzw. immunstimulatorischen Effekten oder Reaktionen auf. "Unspezifisch" bedeutet in diesem Zusammenhang, dass die Auslösung dieser Effekte nicht auf die Sequenz des in der Gentherapie eingesetzten Nukleinsäuremoleküls zurückgeht. Vielmehr werden die immunstimulatorischen Effekte völlig unabhängig von der Sequenz und Struktur des Nukleinsäuremoleküls ausgelöst und treten allein aufgrund des Vorliegens des Nukleinsäuremoleküls auf, sind also einzig auf die Natur und Struktur des Nukeinsäuremoleküls als solches zurückzuführen.

Unter "Nebenwirkungen in Form von immunstimulatorischen Reaktionen oder Effekten" ist eine Auslösung oder Verstärkung einer Immunantwort zu verstehen, die eigentlich nicht im Rahmen der entsprechenden Gentherapie gewünscht bzw. gewollt ist und nicht mit der eingebrachten Sequenz in Verbindung steht. So ist es bei der Verwendung von Nukleinsäuren bekannt, dass Zellen, sobald diese in Kontakt mit bestimmten freien Nukleinsäuren und Nukleinsäurefragmenten kommen, unspezifische zelluläre Reaktionen aufweisen. Diese Reaktionen zeigen sich insbesondere durch immunstimulatorische Effekte wobei beispielsweise oft eine Toll-like-Rezeptor-(TLR)-Aktivierung stattfindet. So ist es beispielsweise bekannt, dass das Gen IFIT1/2 (Interferon induced-protein with tetratricopeptid repeats1/2) durch dsRNA sowie durch INFa/b induziert werden kann. Das codierte Protein IFIT1/2 erkennt dann wiederum ssRNA-Moleküle.

Das Gen IRF9 (Interferon regulatory factor 9) wird durch INFa/b, indirekt durch Nukleinsäuren, induziert und IRF9 selbst ist Bestandteil des INF Transkriptionsaktivatorkomplexes.

Ferner ist bekannt, dass TLR3 (Toll-like receptor 3) dsRNA erkennt, während TLR7 (Toll-like receptor 7) ssRNA und TLR8 G-reiche Oligonucleotide erkennt. PKR (Protein kinase RNA-activated; auch als protein kinase R (PKR) bekannt), wird durch dsRNA und durch Interferon induziert.

Somit kann also die (unspezifische) Aktivierung der oben genannten Gene IFIT1/2, IRF9, TLR3, TLR7, TLR8 und/oder PKR als Indikator verwendet werden, ob immunstimulatorische Reaktionen/immunstimulatorische Effekte im Sinne der vorliegenden Erfindung durch ein in der Gentherapie eingesetztes Nukleinsäuremolekül auftreten.

Als Folge der Aktivierung dieser immunstimulatorischen Reaktionen oder Effekte kommt es direkt oder indirekt zur Expression von Interferonen, die Folgeprozesse in Zellen auslösen, die generell ungewünscht sind und mit Nebenwirkungen verbunden sind, da damit eine allgemeine Expressionshemmung und/oder eine Zytotoxizität einhergeht. In *vivo* können diese zellulären Prozesse erhebliche Folgen haben, und sich beispielsweise in Form von Rötungen, Entzündungen, grippeähnliche Symptome und/oder Fieber äußern.

Wie bereits erwähnt, liegt der vorliegenden Erfindung zugrunde, dass gefunden wurde, dass durch die Bindung eines Peptids an ein in der Gentherapie eingesetztes Nukleinsäuremolekül, bevorzugt eine siRNA, potentiell auftretende (unspezifische) immunstimulatorische Reaktionen/Effekte reduziert bzw. eliminiert werden kann. Die Reduktion der TLR- bzw. Interferon-Aktivierung von siRNA durch Peptidbindung ist besonders deshalb von Nutzen, weil dadurch beispielsweise das Auftreten unspezifischer, nicht auf Grundlage von RNA-Interferenz induzierter Effekte und Phenotypen vermindert werden können. In vivo können hierdurch unspezifische Effekte, die beispielsweise durch siRNA-Moleküle ausgelöst werden, vermindert oder eliminiert werden. Dadurch können dann auch solche Sequenzen genutzt werden, die zwar hochgradig spezifisch für eine bestimmte mRNA sind, aufgrund ihrer unspezifischen immunstimulatorischen Wirkung hingegen aber starke unspezifische Effekte und Phenotypen induzieren, weshalb diese in vivo und in vitro nicht oder nur eingeschränkt nutzbar sind, da deren Nutzung starke Nebenwirkungen induzieren würde.

Unter "Reduktion" von Nebenwirkungen in Form von immunstimmulatorischen Effekten oder Reaktionen ist eine Behandlung zu verstehen, die darauf abzielt, einen gewünschten pharmakologischen und/oder physiologischen Effekt zu erzielen. Um genauer zu sein, versteht sich die Behandlung zur "eduktion" von Nebenwirkungen in Form von immunstimmulatorischen Effekten oder Reaktionen so, dass die Krankheiten und/oder Nebenwirkungen, die mit den immunstimulatorischen Effekten einhergehen, teilweise oder, bevorzugt, vollständig geheilt werden, oder aber, dass diese immunstimulatorischen Effekte oder Reaktionen gar nicht erst auftreten.

Die Behandlung zur "Reduktion" von Nebenwirkungen in Form von immunstimmulatorischen Effekten oder Reaktionen kann einerseits prophylaktisch sein, oder andererseits eine akute Behandlung sein. Im Rahmen der vorliegenden Erfindung ist es bevorzugt, dass die "Reduktion" von Nebenwirkungen in Form von immunstimmulatorischen Effekten oder Reaktionen bei der Verabreichung des in der Gentherapie eingesetzten Nukleinsäuremoleküls gar nicht erst auftreten. Somit wirkt sich die Reduktion der Nebenwirkungen bevorzugt zeitgleich mit der Verabreichung des in der Gentherapie eingesetzten Nukleinsäuremoleküls aus bzw. tritt gar nicht erst (oder nur vermindert) auf. Dies erfolgt, wie oben dargelegt, durch die erfindungsgemäße Kopplung eines Peptids an das in der Gentherapie eingesetzten Nukleinsäuremoleküls.

Somit betrifft die vorliegende Erfindung in einer Ausführungsform ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist, wobei die immunstimulatorischen Reaktionen/Effekte ausgelöst werden durch eine Aktivierung von IFIT1/2, IRF9, TLR3, TLR7, TLR8 oder PKR. Mit der Aktivierung von IFIT1/2, IRF9, TLR3, TLR7, TLR8 oder PKR ist gemeint, dass die Expression dieser genannten Gene aktiviert wird. Wie oben bereits erwähnt, ist es bekannt, dass diese Gene (unspezifisch, unabhängig von der Struktur und/oder Sequenz des Nukleinsäuremoleküls) aktiviert werden, wenn (freie) Nukleinsäuremoleküle oder Fragmente davon in der Zelle auftreten. Deshalb kann die (unspezifische) Aktivierung dieser Gene IFIT1/2, IRF9, TLR3, TLR7, TLR8 und/oder PKR als Indikator verwendet werden, ob immunstimulatorische Reaktionen/immunstimulatorische Effekte im Sinne der vorliegenden Erfindung durch ein in der Gentherapie eingesetztes Nukleinsäuremolekül auftreten, denn diesen immunstimulatorischen Reaktionen/immunstimulatorischen Effekten liegt die Aktivierung dieser Gene bzw. des IFIT1/2-Pathways, IRF9-Pathways, TLR3-Pathways, TLR7-Pathways, TLR8-Pathways und/oder PKR-Pathways zu Grunde.

Dem Fachmann sind Verfahren bekannt, mit denen festgestellt werden kann, ob ein Nukleinsäuremolekül immunstimulatorische Effekte oder Reaktionen auslöst, die durch eine Aktivierung der genannten Gene, also des IFITl/2-Pathways, IRF9-Pathways, TLR3-Pathways, TLR7-Pathways, TLR8-Pathways oder PKR-Pathways gekennzeichnet sind, so dass der Fachmann klar erkennen und entscheiden kann, ob Nebenwirkungen, die bei einer Gentherapie (durch das eingesetzte Nukleinsäuremolekül) auftreten, auf die Aktivierung des IFIT1/2-Pathways, IRF9-Pathways, TLR3-Pathways, TLR7-Pathways, TLR8-Pathways und/oder des PKR-Pathways zurückzuführen sind. Entsprechend kann der Fachmann dann mit denselben Verfahren auch feststellen, ob die Kopplung an ein Peptid diese Nebenwirkungen in Form von immunstimulatorischen Reaktionen/Effekten reduzieren kann.

Bekannte Verfahren zur Bestimmung, ob IFIT1/2, IRF9, TLR3, TLR7, TLR8 oder PKR aktiviert werden, sind im Folgenden jeweils kurz beschrieben.

Ob IFIT1/2, IRF9, TLR3, TLR7, TLR8 bzw. PKR aktiviert wird, und somit ob eine entsprechende Immunstimulierung stattfindet, lässt sich beispielsweise durch die Analyse der Genexpression von IFIT1/2, IRF9, TLR3, TLR7, TLR8 bzw. PKR untersuchen, denn mit der Aktivierung dieser Gene ist stets eine Neuexpression oder eine Erhöhung der Expression dieser Gene verbunden. Somit können immunstimulatorische Reaktionen/Effekte nachgewiesen werden, wenn eine Neuexpression von IFIT1/2, IRF9, TLR3, TLR7, TLR8 bzw. PKR festgestellt wird. Dem Fachmann sind viele Verfahren bekannt, wie festgestellt werden kann, ob ein Gen exprimiert wird. So wird häufig eine quantitative PCR eingesetzt, bei der zunächst aus den zu untersuchenden Zellen oder Geweben die mRNA isoliert wird. Über eine reverse Transkription wird die mRNA in eine cDNA umgeschrieben. Mittels geeigneter Primer, die spezifisch für IFIT1/2, IRF9, TLR3, TLR7, TLR8 bzw. PKR sind, kann die DNA über eine PCR-Reaktion amplifiziert werden, wobei aus der Amplifizierung der DNA des zu untersuchenden Gens im Vergleich zu einem Referenzgen die relative oder, in Abhängigkeit zu einem DNA-Standard, die absolute Menge an Ausgangs-mRNA des zu untersuchenden Gens berechnet werden kann.

Dem Fachmann sind solche Verfahren zur Quantifizierung von mRNA bekannt, über die die Neuexpression oder Erhöhung der Expression von IFIT1/2, IRF9, TLR3, TLR7, TLR8 bzw. PKR bestimmt werden kann. Andere Verfahren bestimmen die Expression nicht auf mRNA-Ebene sondern weisen die Neuexpression oder die Erhöhung der Expression von IFIT1/2, IRF9, TLR3, TLR7, TLR8 bzw. PKR auf Protein-Ebene nach. Beispielsweise kann die (Erhöhung bzw. Neusynthese) der Proteinmenge von IFIT1/2, IRF9, TLR3, TLR7, TLR8 bzw. PKR über eine Western-Blot-Analyse nachgewiesen werden.

Immunstimulatorische Reaktionen/Effekte in Übereinstimmung mit dem Vorstehenden liegen dann vor (oder werden dann festgestellt), wenn die Expression von IF1T1/2, IRF9, TLR3, TLR7, TLR8 und/oder PKR durch die Gegenwart von dem in der Gentherapie eingesetzten Nukleinsäuremolekül in den oben genannten Testverfahren bevorzugt um mindestens das 1,2 fache, 1,5 fache oder 2 fache gegenüber einer Kontrolle ohne das in der Gentherapie eingesetzte Nukleinsäuremolekül erhöht ist. In einer weiteren Ausführungsform liegen immunstimulatorische Reaktionen/Effekte dann vor oder werden dann festgestellt, wenn die Expression von IFIT1/2, IRF9, TLR3, TLR7, TLR8 und/oder PKR durch die Gegenwart von dem in der Gentherapie eingesetzten Nukleinsäuremolekül in den oben genannten Testverfahren um mindestens das 2,5 fache, 5 fache oder 10 fache gegenüber einer Kontrolle ohne das in der Gentherapie eingesetzte Nukleinsäuremolekül erhöht ist. In einer weiteren Ausführungsform liegen immunstimulatorische Reaktionen/Effekte dann vor oder werden dann festgestellt, wenn die Expression von IFIT1/2, IRF9, TLR3, TLR7, TLR8 und/oder PKR durch die Gegenwart von dem in der Gentherapie eingesetzten Nukleinsäuremolekül in den oben genannten Testverfahren um mindestens das 15 fache, 20 fache, 30 fache oder 50 fache gegenüber einer Kontrolle ohne das in der Gentherapie eingesetzte Nukleinsäuremolekül erhöht ist. Im Stand der Technik wird eine 15 fache Erhöhung der Expression als kritischer Wert angesehen, ab der eine immunstimulatorische Reaktion/ein immunstimulatorischer Effekt vorliegt (Dorn, A., et al., J. Virol. Feb;79(4):2404-12 (2005)). Somit liegen in einer bevorzugten Ausführungsform immunstimulatorische Reaktionen/Effekte dann vor oder werden dann festgestellt, wenn die Expression von IFIT1/2, IRF9, TLR3, TLR7, TLR8 und/oder PKR durch die Gegenwart von dem in der Gentherapie eingesetzten Nukleinsäuremolekül in den oben genannten Testverfahren um mindestens das 15 fache, gegenüber einer Kontrolle ohne das in der Gentherapie eingesetzte Nukleinsäuremolekül, erhöht ist.

In einer weiteren Ausführungsform betrifft die vorliegende Erfindung ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist, wobei die immunstimulatorischen Reaktionen/Effekte zu Rötungen, Entzündungen, grippeähnlichen Symptomen und/oder Fieber führen.

Es ist beschrieben, dass das Peptid, das an ein Nukleinsäuremolekül gekoppelt ist, durch Enzyme spezifisch abspaltbar ist. Im Stand der Technik sind derartige Nukleinsäuremoleküle beschrieben, die an für bestimmte Proteasen spezifische Peptide gekoppelt sind. Auch sind Verfahren beschrieben, wie man diese Peptide an Nukleinsäuremoleküle koppeln kann. Beispielsweise ist es beschrieben, dass durch Bindung kurzer Peptide, welche zellspezifisch abgespalten werden, eine definierte Zellspezifität erreicht werden kann (WO 2008/098569 A2). Am Beispiel von siRNA Molekülen ist es gezeigt worden, dass durch den Einsatz derart modifizierter siRNA Moleküle selektiv in bestimmten Zellen die Expression von Genen reduziert bzw. unterbunden werden kann, während in anderen Zellen, die nicht über entsprechende zellspezifische Proteasen verfügen, die Aktivität dieser siRNA Moleküle nicht freigesetzt wird, da die Peptide (hemmend) an den siRNA Molekülen gebunden bleiben.

Die Abspaltung durch spezifische Enzyme kann insbesondere dadurch induziert werden, dass diese spezifischen Enzyme beispielsweise (nur) bei spezifischen Krankheits- oder Entwicklungszuständen von Zellen (insbesondere Zellzyklus oder Ausdifferenzierung bei Stammzellen) aktiv sind. Auch können diese Enzyme spezifisch für bestimmte Zellarten oder krankheitsrelevante Veränderung der Zellen (insbesondere bei Entartung oder Infektion) sein. Auch können die Enzyme genotypspezifisch eine Aktivität zeigen. Des Weiteren kann eine spezifische Abspaltung zur Detektion bestimmter Enzyme oder bei den erwähnten Anwendungen erfolgen.

Spezifische Enzyme können hierbei beispielsweise Proteasen oder Peptidasen (Caspasen, Aminopeptidasen oder Serinproteasen; beispielsweise Caspase-1, Caspase-2, Caspase-3, Caspase-4, Caspase-5, Caspase-6, Caspase-7, Caspase-8, KLK4, PLAP, IRAP, uPA, FAP-α, Legumain oder virale Proteasen, beispielsweise HIV-Protease, Coxsackievirus-Protease, Epstein-Barr-Virus-Protease, Hepatisis-A, -B, -C Virus-Protease), Nukleasen, Glycosidasen, Saccharasen oder Chitinasen sein, wobei dies nicht-limitierende Beispiele sind. Dem Fachmann sind eine Vielzahl spezifischer Peptidsequenzen sowie die entsprechenden Proteasen oder Peptidasen, Nukleasen, Glycosidasen, Saccharasen oder Chitinasen, die diese Peptidsquenzen spezifisch erkennen und spalten können, bekannt.

Auch kann zwischen dem Nukleinsäuremolekül und dem Peptid, das spezifisch abgespalten werden kann, ein Amino-Cn-Linker (mit Cn = Cl, C2, C3, C4, C5 oder C6), beispielsweise ein Amino-C6-Linker, an dem 3'- oder/und 5 '-Ende des Nukleinsäuremoleküls mit dem Peptid kovalent gekoppelt sein. Durch diese kovalente Bindung kann das Nukleinsäuremolekül, bevorzugt das siRNA Molekül inaktiviert werden. Handelt es sich bei dem Nukleinsäuremokelül um eine siRNA, findet somit nach einer Transfektion solcher inaktiven siRNA Moleküle keine Hemmung einer spezifischen Genexpression statt, solang auch nur eine der gebundenen Peptide durch das Nichtvorhandensein des entsprechenden zielzellentypischen Enzyms an den siRNA-Molekülen verbleibt. Zur Aktivierung der Nukleinsäuremoleküle, bevorzugt der siRNA, kann dann das Peptid von der siRNA abgespalten werden, wobei der Amino-Cn-Linker und eventuell ein Peptidrest an der siRNA verbleibt. Derartige Linker sind beispielsweise in WO2010/102615 beschrieben.

Beschrieben ist auch hierin ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist, wobei das Peptid unspezifisch, bevorzugt durch pH-Wert-Änderung, abspaltbar ist. Entsprechende Verfahren sind im Stand der Technik bekannt und beschrieben (siehe beispielsweise Kun Cheng and Ram I. Malxato "Advanced Delivery and Therapeutic Applications of RNAi"; Wiley publishing group (2013)). Darin sind beispielsweise in Abschnitt 6.2.3.3 enzymatisch spaltbare Linker und in Abschnitt 6.2.3.4 säurelabile Linker beschrieben.

In einer weiteren Ausfiihrungsform betrifft die vorliegende Erfindung ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist, wobei das Nukleinsäuremolekül weitere chemische Modifikationen umfasst. Beispielsweise kann das Peptid, das an das Nukleinsäuremolekül gekoppelt ist, oder aber direkt das Nukleinsäuremolekül, das in der Gentherapie eingesetzt wird, an Moleküle gebunden sein, z.B. für einen besseren Transport in bzw. an die Zellen (beispielsweise Nanopartikel als Trägersystem) sowie zur Stabilisierung oder zur Detektion (beispielsweise Fluorochrome zur Detektion).

Wie bereits oben dargelegt, sind dem Fachmann Verfahren bekannt, wie das Nukleinsäuremolekül, das in der Gentherapie eingesetzt wird, (spezifisch) an den Zielort, d.h. an oder in eine gewünschte Zielzelle, gelangen kann. Dazu können die erfindungsgemäßen Peptid/Nukleinsäuremolekül-Komplexe der vorliegenden Erfindung beispielsweise in an sich bekannter Weise über Delivery Mechanismen in Zellen eingebracht werden. Die verwendeten Delivery-Mechanismen können eine Zell-Selektivität aufweisen und entweder ungebunden/komplexierend oder ebenfalls kovalent an die Nukleinsäuren gebunden sein. Die Peptide selbst können ebenfalls als Delivery-Mechanismus fungieren oder den Delivery-Prozess in der Gesamtheit (Eintritt in die Zelle und Übergang in das Zytoplasma) oder als Teilprozess (z.B. Ausschleusung aus dem Endosom in das Zytoplasma) unterstützen.

Durch ein geeignetes Transfektionssystem, beispielsweise Nanopartikel, Polyethylenimin oder Liposomen, können die erfindungsgemäßen Peptid/Nukleinsäuremolekül-Konstrukte in gleichfalls an sich bekannter Weise in die Zellen eingebracht werden.

Die Natur des Peptids wie hier beschrieben, das an ein in der Gentherapie eingesetzten Nukleinsäuremolekül gekoppelt ist, ist nicht auf ein spezifisches Peptid oder eine spezifische Peptidsequenz limitiert. Vielmehr konnte, wie oben bereits erwähnt, in den Beispielen gezeigt werden, dass die Kopplung eines Peptids einen Einfluss auf die unspezifischen Effekte hat, die sonst die bei der Verwendung von Nukleinsäuren im Rahmen von Gentherapien auftreten, und zwar unabhängig von der Natur des eingesetzten Peptids. Wie erwähnt, konnte gezeigt werden, dass diese (unspezifischen) immunstimulatorischen Effekte/Reaktionen drastisch durch die Bindung eines Peptids reduziert werden können. Diese Reduktion ist losgelöst von der Struktur oder der Sequenz des eingesetzten Peptids. Die Natur des Peptids scheint also nicht wesentlich zu sein. Vielmehr kann deshalb ein Peptid mit beliebiger Sequenz und beliebiger Länge erfindungsgemäß eingesetzt werden.

Ein erfindungsgemäß verwendetes Peptid ist eine organische Verbindung, die Peptidbindungen zwischen Aminosäuren enthält und als ein kleines Protein betrachtet werden kann. Dabei sind die einzelnen Aminosäuren in einer definierten Reihenfolge (Sequenz) meist linear zu einer Kette verbunden, können aber auch verzweigt oder zirkulär vorliegen. Eine Peptidbindung ist eine amidartige Bindung zwischen der Carboxygruppe einer Aminosäure und der Aminogruppe des α-Kohlenstoffatoms (α-C-Atom) einer zweiten Aminosäure. Zwei Aminosäuren können (formal) unter Wasserabspaltung zu einem Dipeptid kondensieren. Durch mehrfache Kondensation bilden sich Tripeptide, Tetrapeptide, Oligopeptide und schließlich Polypeptide. Peptide sind meist kettenförmige, aus Aminosäuren aufgebaute Makromoleküle. Peptide unterscheiden sich von Proteinen vor allem durch ihre molaren Massen, das heißt durch die Anzahl der verknüpften Aminosäuren. Die Abgrenzung ist fließend; bei mehr als ungefähr 100 verknüpften Aminosäuren wird das Molekül als Protein bezeichnet, kann aber auch noch im Zusammenhang der vorliegenden Beschreibung als Peptid verstanden werden. Ein

Peptid kann aus 2 Aminosäuren bestehen (Dipeptid), aus 3 Aminosäuren, (Tripeptid), aus 4 Aminosäuren (Tetrapeptid), oder aus 5 Aminosäuren (Pentapeptid). Bevorzugt besteht das Peptid aus bis zu 10 Aminosäuren, also zwischen 2 und 10 Aminosäuren und wird dann Oligopeptid genannt. Es ist zudem beschrieben, dass das Peptid zwischen 10 und 100 Aminosäuren, also aus bis zu 15, 20, 30, 40, 50, 60, 70, 80, 90 oder 100 Aminosäuren bestehen kann und wird dann Polypeptid genannt. In einer weiteren Ausführungsform kann das Peptid aber auch mehr als 100 Aminosäuren haben, beispielsweise bis zu 150, 200 oder 300 Aminosäuren. In einer weiteren bevorzugten Ausführungsform besteht das Peptid aus bis zu 12 Aminosäuren, bevorzugt zwischen 4 und 12 Aminosäuren.

Wie bereits erwähnt, ist die Sequenz des Peptids nicht wesentlich für die vorliegende Beschreibung. So kann jede (unspezifische) Aminosäuresequenz als Peptid, wie hierin beschrieben, verwendet werden. Es können aber spezifische Peptidsequenzen gewählt werden, um beispielsweise eine spezifische Abspaltung des Peptids von dem in der Gentherapie eingesetzten Nukleinsäuremolekül zuermöglichen. Dazu entspricht die Peptidsequenz einer Spaltsequenz für entsprechende Peptidasen. Wie oben bereits erwähnt, sind derartige Konstrukte im Stand der Technik beschrieben. So sind Methoden beschrieben, mit denen durch Bindung kurzer Peptide, welche zellspezifisch abgespalten werden, eine Zellspezifität erreicht werden kann (WO 2008/098569 A2). Dies ist insbesondere dann vorteilhaft, wenn siRNA Moleküle eingesetzt werden. Durch die Bindung von entsprechenden Peptidsequenzen an das Nukleinsäuremolekül, das in der Gentherapie eingesetzt wird, bevorzugt an ein siRNA Molekül, kann erreicht werden, dass selektiv in bestimmten Zellen die Expression von Genen reduziert bzw. unterbunden wird. Entsprechende spezifische Sequenzen (sowie die entsprechenden Peptidasen, die diese Sequenzen erkennen und spalten können) sind dem Fachmann bekannt und die Sequenz des erfindungsgemäßen Peptids, das an ein in der Gentherapie eingesetztes Nukleinsäuremolekül gekoppelt ist, ist nicht auf eine spezifische Peptidsequenz limitiert. Wie in den Beispielen gezeigt, wurden Peptide in einer Länge von 4 bis 12 Aminosäuren mit bekannten Schnittstellen (oder neue, nicht im Stand der Technik beschriebenen Schnittstellen) für zelluläre Proteasen getestet.

Somit können die gekoppelten Peptide des Weiteren Zielsequenzen von Proteasen enthalten und/oder in Form von ebenfalls bekannten Prodrug-Anwendungen gezielt in Zielzellen aktiviert werden. Unter Prodrug-Anwendungen versteht man die Möglichkeit, die durch die Nukleinsäuremoleküle ausgelöste Immunstimulation gezielt zu nutzen, um beispielsweise Zellen gezielt absterben zu lassen. Dies kann mit den erfindungsgemäßen Peptid/Nukleinsäuremolekül-Konstrukten erreicht werden, indem diese so gestaltet sind, dass durch eine gezielte Abspaltung des Peptids in bestimmten Zielzellen das Immunsystem lokal stimuliert wird. Es ist bekannt, dass der Organismus das Immunsystem gezielt nutzt und aktiviert, wenn einzelne Zellen absterben sollen. Dies kann beispielsweise durch Verwendung der erfindungsgemäßen Peptid/Nukleinsäuremolekül-Konstrukten erreicht werden, damit eine Virus-infizierte Wirtszelle gezielt und sicher abstirbt. Ursächlich für das gezielte Absterben ist es, dass in der Zielzelle das vor Immunreaktionen schützende Peptid selektiv von dem Nukleinsäuremolekül, beispielsweise der siRNA, abgespalten wird und dadurch in der Zielzelle eine (an sich durch herkömmliche Nukleinsäuren auftretende) TLR-vermittelte Stressreaktion entsteht. Durch den Zellstress wird die Zelle abgetötet und dadurch virale Proteine und DNA/RNA des Virus frei, worauf dann das Immunsystem wiederum (lokal) reagiert. Außerdem tragen die nun frei gewordenen erfindungsgemäßen Nukleinsäuremoleküle nach der Abspaltung der Peptide zusätzlich zu einer zellulären Immunstimulation (beispielswiese durch T-Zellen) bei. Hierdurch wird das Immunsystem lokal stimuliert. Erfindungsgemäß kann also die Peptidkopplung genutzt werden, um einerseits den immunstimulierenden Effekt der Nukleinsäuremoleküle (beispielsweise in nicht-Virus-infizierten Zellen) zu inhibieren, um diese dann gezielt in einigen Zellen oder Zellpopulationen (und nur dort) durch Abspaltung des Peptids freizusetzen und die durch (freie) Nukleinsäuremoleküle ausgelösten immunstimulierenden Effekte hervorzurufen. Dies kann beispielsweise in Virus-infizierten Zellen erfolgen, um das Immunsystem in den betroffenen Geweben (unterstützend) zu aktivieren, während in anderen, nicht-infizierten Zellen das Peptid nicht abgespalten wird. Im Stand der Technik ist beschrieben, wie durch Bindung kurzer Peptide, welche zellspezifisch abgespalten werden können, eine definierte Zellspezifität erreicht werden kann (WO 2008/098569 A2). Somit ist es also möglich, die erfindungsgemäße Peptidbindung an das Nukleinsäuremolekül zu nutzen, einerseits gezielt in bestimmten Zellen (durch spezifische Abspaltung des Peptids in diesen Zellen) gewünschte immunstimulierende Effekte auszulösen, während das so freigesetzte Nukleinsäuremolekül, beispielsweise eine siRNA, (sequenzspezifisch) zusätzlich noch auf die Expression der viralen DNA-/RNA-/Protein-Moleküle prozessierend/hemmend/inhibierend, beispielswiese über den Mechanismus der RNA Interferenz, einwirken kann. In ähnlicher Weise können Peptid-gekoppelte Nukleinsäuremoleküle bei Tumorerkrankungen eingesetzt werden, bei denen normalerweise das Immunsystem nicht aktiviert wird bzw. Zellen des Immunsystems die Tumorzellen nicht erkennen. Systemisch verabreichte erfindungsgemäße Peptid/Nukleinsäuremoleküle können dann so gestaltet sein, dass die Peptide nur in Tumorzellen abgespalten werden, mit dem Ziel, gezielt in Tumorzellen eine lokale Immunstimulation durch die freien Nukleinsäuremoleküle auszulösen. Beide Mechanismen zur Induktion von Zellspezifität können auch kombiniert zur Anwendung kommen.

Weiterhin ist ein Peptid zur Verwendung in der Reduktion von Nebenwirkungen in Form von immunstimulatorischen Reaktionen/Effekten, die bei einer Gentherapie auftreten beschrieben, wobei das Peptid an ein in der Gentherapie eingesetztes Nukleinsäuremolekül, bevorzugt ein siRNA Molekül, gekoppelt ist, wobei das Peptid die Spaltsequenz für Legumain (Spaltsequenz: Ala-Ala-Asn), Capase-4 (Spaltsequenz: Leu-Glu-Val-Asp-Gly; SEQ ID NO:1), uPA (Spaltsequenz: Glu-Gly-Arg), Fap (Spaltsequenz: Gly-Pro), KLK (Spaltsequenz: Val-Gln-Gln-Lys-Ser; SEQ ID NO:2), PLAP (Spaltsequenz: Pro-His-Ile-Tyr-Val, SEQ ID NO:3), Cathepsin-E (Spaltsequenz: Gly-Gly-Ala-Phe-Leu-Val-Leu-Pro; SEQ ID NO:4; bzw. Spaltsequenz: Ala-Leu-Ala-Phe-Ser-Leu-Ala-Ala; SEQ ID NO:5), CMV-Protease (Spaltsequenz: Pro-Ser-Val-Ser-Ala; SEQ ID NO:6), oder EBV-Protease (Spaltsequenz: Gly-Ser-Ala-Ser-Ala, SEQ ID NO:7) umfasst.

Die Lokalisierung des Peptids spielt dabei keine Rolle. Das Peptid kann entweder an das 5'-Ende und/oder an das 3'-Ende des Nukleinsäuremoleküls gekoppelt werden. Handelt es sich bei dem Nukleinsäuremolekül um eine siRNA, so ist die beschriebene Bindung von Peptiden nicht an die 5'-Position des Rückgrates des Antisense-Stranges von siRNA beschränkt. Zur Peptidbindung kann beispielsweise auch die 3'-Position des Nukleinsäure-Rückgrates, eine Bindung an der Base oder am Rückgrat zwischen zwei Nukleotiden genutzt werden. Im Fall von doppelsträngigen Nukleinsäuren kommt auch der Sense-Strang in Frage. Im Fall von Nukleinsäure-Analoga ist ebenfalls die Bindung von Peptiden an die Enden, den Basen oder das Rückgrat möglich. Dem Fachmann sind Verfahren bekannt, wie Peptide an Nukleinsäuremoleküle gekoppelt werden können.

Das hierin beschriebene Peptid, das an ein Nukleinsäuremolekül gekoppelt ist, ist auf die spezifische Spaltsequenz für Legumain (Spaltsequenz: Ala-Ala-Asn), Insbesondere kann das Peptid auch wie im Folgenden beschrieben, modifiziert sein, denn in einer weiteren Ausführungsform ist das hierin beschriebene Peptid, das an ein Nukleinsäuremolekül gekoppelt ist, auf die spezifische Spaltsequenz für Legumain (Spaltsequenz: Ala-Ala-Asn),

Es ist beschrieben, dass die Spaltsequenz eine Sequenz, die eine oder mehrere Hinzufügung(en) von Aminosäuren an der Sequenz Ala-Ala-Asn umfasst die ausgewählt ist aus der Gruppe bestehend aus Ala-Ala-Asn, SEQ ID NO:1, Glu-Gly-Arg, Gly-Pro, SEQ ID NO:2, SEQ ID NO:3, SEQ ID NO:4; SEQ ID NO:5, SEQ ID NO:6, UND SEQ ID NO:7, und die durch die entsprechende Protease, also durch Legumain, Capase-4, uPA, Fap, KLK, PLAP, Cathepsin-E, CMV-Protease, oder EBV-Protease, gespaltet werden kann. Die Hinzufügung von Aminosäure(n) kann entweder am N- oder C-terminalen Ende des Peptids sein. Die hinzugefügten Aminosäure(n) umfassen bis zu 0 (keine Hinzufügung), 1, 2, 3, 4, 5, 6, 7, 8, 9 oder 10 Aminosäuren, bevorzugt bis zu 20 Aminosäuren oder mehr, bevorzugt bis zu 30 Aminosäuren. Da die Hinzufügung von Aminosäuren generell die oben genannten funktionellen Eigenschaften der Spaltbarkeit der Peptide wenig oder gar nicht beeinflusst, können die hinzugefügten Aminosäuren eine Länge von bis zu 40, 50, 60, 70, 80, 90, oder 100 Aminosäuren und mehr, bis zu 200, 300, 400 oder 500 Aminosäuren haben.

Dem Fachmann sind Verfahren bekannt, wie Peptide synthetisiert werden können. So kann das Peptid der vorliegenden Erfindung rekombinant hergestellt werden, oder synthetisch durch (chemische) Peptidsynthese. Auch kann der Fachmann problemlos einzelne oder mehrere Aminosäuren in einer Sequenz durch gezielte Mutagenese verändern, um zu den oben genannten Modifikationen zu gelangen.

Wie bereits erwähnt, wird das erfindungsgemäße Peptid an das in der Gentherapie eingesetzte Nukleinsäuremolekül gekoppelt. Dem Fachmann sind Verfahren bekannt, wie Peptide an Nukleinsäuremoleküle gekoppelt werden können. Dies kann beispielsweise über eine Peptidbindung erfolgen. Als Peptidbindung wird die chemisch-kovalente Anbindung von Molekülen an die Nukleinsäuren verstanden, welche mindestens eine Peptidbindung enthalten. Wie bereits oben erwähnt, kann die Anbindung über Linker-Moleküle erfolgen. Diese Linker-Moleküle können durch äußere Einflüsse aufbrechbar sein (beispielsweise durch pH-Wertänderung) oder enzymatisch spaltbar sein.

Wie im folgenden noch näher erläutert wird ist das Nukleinsäuremolekül, das an ein Peptid im Sinne der Erfindung gekoppelt ist, nicht auf ein spezifisches Nukleinsäuremolekül oder auf eine spezifische Nukleinsäuremolekül-Spezies beschränkt und die erfindungsgemäße Peptidkopplung kann beispielsweise bei siRNA, shRNA, saRNA, miRNA oder weiteren RNA-Formen, sowie in Form von DNA, PNA oder weiterer Nukleotid-Analoga in der herkömmlichen oder chemisch modifizierten Form mit oder ohne räumlicher Struktur, Aptameren oder RNA/DNA-Mischsequenzen erfolgen.

In Übereinstimmung mit dem Vorstehenden kann das Peptid, zur Verwendung in der Reduktion von Nebenwirkungen in Form von immunstimulatorischen Reaktionen/Effekten, die bei einer Gentherapie auftreten, somit auch beispielsweise bei kurzen Nukleinsäuren (beispielsweise saRNA) oder langen Nukleinsäuren (beispielsweise miRNA, mRNA, Dicer-Substraten, PNA und DNA) eingesetzt werden. Die genannten Nukleinsäuren stellen eine Auswahl dar, die Erfindung ist aber nicht auf diese beschränkt. Als Nukleinsäuren im Sinne der Erfindung werden einzel- oder doppelsträngige RNA, DNA, RNA-DNA-Mischsequenzen, chemisch modifizierte oder unmodifizierte RNA/DNA, mit oder ohne Einzelstrang-Überhänge, DNA oder RNA mit räumlicher Struktur, Dicer Substrate oder Aptamere, sowie Nukleinsäure-Analoge (beispielsweise PNA) verstanden. Außerdem können an die Nukleinsäuren weitere Moleküle gebunden sein, um eine Funktionalität (Tracer, Analytik, Zell-Targetting, Endosomal Escape, Zellpenetrierung, Serumstabilität, Nukleasestabilität, u.a.) zu erreichen.

Deshalb betrifft die vorliegende Erfindung in einer weiteren Ausführungsform ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül gekoppelt ist, wobei das Nukleinsäuremolekül ausgewählt ist aus der Gruppe bestehend aus mRNA, siRNA, miRNA, saRNA, RNA, PNA, DNA, LNA, Gemischen aus verschiedenen Nukleotiden und Aptameren. Das erfindungsgemäße Koppeln von Peptiden an einzelne Nukleotide (die ggf. in Gemischen vorliegen können) ist dann beispielsweise vorteilhaft, wenn diese Nukleotide in einem größeren Molekül, z.B. einem Polymer-Komplex eingebunden sind, um beispielsweise einen Ladungsausgleich zu schaffen. Einzelne, in einem solchen Polymer-Komplex eingebaute Nukleotid-Moleküle können dann eine ungewünschte Immunreaktion auslösen, wovor die erfindungsgemäße Kopplung an ein Peptid schützen kann.

In einer bevorzugten Ausfiihrungsform ist das Nukleinsäuremolekül eine siRNA.

Wie bereits oben erwähnt ist die Natur des Nukleinsäuremoleküls nicht auf bestimmte Nukleinsäuremolekül-Spezies limitiert und kann jedes, in der Gentherapie eingesetztes, Nukleinsäuremolekül sein. So kann das erfindungsgemäße einzel- oder doppelsträngige Nukleinsäuremolekül eine mRNA, siRNA, miRNA, saRNA, RNA, PNA, DNA, LNA, Gemischen aus verschiedenen Nukleotiden und Aptameren sein. mRNA, siRNA, miRNA, saRNA, RNA, PNA, DNA und LNA sind dem Fachmann bekannt. Das erfindungsgemäße Nukleinsäuremolekül kann also ein einzel- oder doppelsträngiges Nukleinsäuremolekül sein. Es kann aber auch ein Aptamer oder ein Spiegelmer sein. Außerdem wird das erfindungsgemäße Nukleinsäuremolekül nicht nur in Form einer der vorgenannten einzelnen Nukleinsäure-Spezies bereitgestellt. Vielmehr werden in einer bevorzugten Ausführungsform auch Mischungen oder Mischformen aus den einzelnen Spezies (mRNA, siRNA, miRNA, saRNA, RNA, PNA, DNA, LNA, Aptamer und/oder Spiegelmer) verwendet, die erfindungsgemäß an ein Peptid gekoppelt sind.

Der Begriff "Aptamer" umfasst kurze einzelsträngige DNA- oder RNA-Oligonukleotide, die in der Lage sind, ein spezifisches Molekül über ihre dreidimensionale Struktur zu binden. Der Begriff "Spiegelmer" umfasst L-Ribonukleinsäureaptamere (kurz L-RNA-Aptamere). L-Ribonukleinsäureaptamere sind der Ribonukleinsäure (RNA) ähnliche Moleküle, die aus unnatürlichen L-Ribonukleotiden aufgebaut sind. Sie sind künstliche Oligonukleotide und stereochemische Spiegelbilder natürlicher Oligonukleotide. L-Ribonukleinsäureaptamere stellen somit eine spezielle Form der Aptamere dar und können wie diese spezifische Moleküle über ihre dreidimensionale Struktur binden. L-Ribonukleinsäureaptamere sind unter dem Markennamen "Spiegelmer" bekannt.

Wie bereits erwähnt, wird in einer bevorzugten Ausführungsform das Nukleinsäuremolekül in Form einer siRNA bereitgestellt.

Der Einsatz von siRNA in der Gentherapie stellt einen bevorzugten molekularbiologischen Ansatz dar, Zellen gezielt zu beeinflussen. Dabei werden kurze, doppelsträngige RNA-Moleküle eingesetzt. Diese so genannten siRNA (engl. short interfering RNA) Moleküle können klassischer Weise nach ihrer Aktivierung mit der mRNA des Zielgens, das es im Rahmen der Gentherapie spezifisch zu inaktivieren gilt, interagieren und bilden zusammen mit speziellen Endoribonukleasen einen RNA-Proteinkomplex mit der Bezeichnung "RISC" (RNA induced silencing complex). Der RISC Komplex bindet an die Target-mRNA, wobei Endonukleasen die Ziel-mRNA schneiden. Auf diese Weise wird die Genexpression verhindert und somit das Entstehen von Zielproteinen gehemmt. Die Hemmung der Genexpression durch Einbringen von kurzen (19-23bp), doppelsträngigen RNA-Molekülen (siRNA) in eukaryotische Zellen, die spezifisch für einen Sequenzabschnitt der mRNA eines Zielgens ist, wurde bereits beschrieben (Elbashir SM et al.: Duplexes of 21-nucleotide RNAs mediate RNA interference in cultured mammalian cells, Nature, 2001 May 24, 411(6836), 494-8; Liu Y et al.: Efficient and isoform-selective inhibition of cellular gene expression by peptide nucleic acids, Biochemistry, 2004 Feb 24, 43(7), 1921-7; US 5,898,031 A; US 7,056,704 B2). Mit Hilfe solcher Moleküle wird nicht das Ablesen eines Gens und die Produktion einer mRNA verhindert, sondern es wird durch die siRNA ein zell eigener Mechanismus initiiert, der die Target-mRNA abbaut. Schließlich wird, wie vorbeschrieben, die Bildung eines spezifischen Proteins unterdrückt, ohne die Expression weiterer Gene zu beeinträchtigen (post-transcriptional gene silencing).

Die Natur oder die Sequenz des siRNA Moleküls, das im Rahmen der vorliegenden Erfindung an ein Peptid gekoppelt wird, zur Verwendung in der Reduktion von Nebenwirkungen in Form von immunstimulatorischen Reaktionen/Effekten, die bei einer Gentherapie auftreten, ist nicht beschränkt. Vielmehr hängt die Wahl der Sequenz des siRNA Moleküls von dem Gen ab, das in der Gentherapie beeinflusst (im Sinne einer Reduktion oder Eliminierung der Expression des entsprechenden Gens über den siRNA Mechanismus wie oben beschrieben) werden soll.

In einer bevorzugten Ausführungsform betrifft die vorliegende Erfindung ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül gekoppelt ist, wobei die siRNA eine Nucleotidsequenz umfasst, die ausgewählt ist aus der Gruppe bestehend aus:
(5-3) UCA UAU UCG ACU UUG GUU GCC (Polo-like Kinase (PLK); SEQ ID NO:8);
(5-3) UCA AAC UCC AUC AUG AUC U (SEQ ID NO:9) und/oder (5-3) UCC AUC AUG AUC UUC UGG A (SEQ ID NO:10) (CHMP);
(5-3) UUC AUA AAC ACA GUU CUC C (SEQ ID NO:11) (PDCD);
(5-3) UCA AAU UGA GGC ACU GUG C (SEQ ID NO:12) (RFWD);
(5-3) UUU CUU CAG AGC AGG AGC A (SEQ ID NO:13), (5-3) AUA CAC ACC CUU UGC CUC A (SEQ ID NO:14) und/oder (5-3) AUU UCA GGC UCA UAU UCC U (SEQ ID NO:15) (ATAP);
(5-3) CAC AAU UCC CAC UUU GAG C (SEQ ID NO:16), (5-3) GUU ACC CAC AAU UCC CAC U (SEQ ID NO:17) und/oder (5-3) UUU CUU CUC UUU GUC UGG G (SEQ ID NO:18) (AGAP); und
(5-3) UAU UCU CCA AAC AAU GUG C (SEQ ID NO:19) (RCHY).

Die vorangestellten siRNA-Moleküle sind spezifisch für die Zielgene PLK, CHMP, PDCD, RFWD, ATAP, AGAP bzw. RCHY. Die Sequenzen wurden so gewählt, dass diese vorteilhafterweise mit einer Nukleotidsequenz an einen einzigen ausgewählten Bereich der jeweiligen mRNA binden, der anhand von Sequenzierungsanalysen statistisch sehr selten einer Mutation unterliegt, und damit auch im Fall erhöhter Mutationsraten im Gesamt-Genom die Zelle ohne erforderliche weitere mRNA-Bindung oder sonstige Zelleinwirkung zuverlässig abtöten, wobei besonders bevorzugt solche Nukleinsäuremoleküle sind, die siRNA-Sequenzen enthalten, die für die Zielgene PLK, CHMP, PDCD, RFWD, ATAP und AGAP spezifisch sind, um deren Expression zu hemmen. Diese besonders bevorzugten Nukleinsäuremoleküle der vorliegenden Erfindung sind oben genannt und näher beschrieben. Es hat sich gezeigt, dass die siRNA-Sequenzen für diese Gene das Abschalten der Expression zu toxischen Effekten führt ohne dass Zellen auch bei längerer Verwendung derselben siRNA Sequenz resistent gegenüber der Behandlung mit den entsprechenden siRNAs wurden. Dies ist besonders deshalb von Nutzen, weil beispielsweise bei der Beeinflussung von Tumorzellen mittels siRNA Mutationen auftreten können, wodurch eine bestimmte siRNA unwirksam wird und dadurch diese Zellen einen Wachstumsvorteil haben und verstärkt Zellteilung auftritt. Dies wurde unter Benutzung bestimmter Sequenzen für die genannten Gene überraschender Weise nicht beobachtet.

Es sind nicht nur Nukleinsäuremoleküle umfasst, die die oben genannten Sequenzen SEQ ID NO:8 bis 19 umfassen. Vielmehr können auch solche Sequenzen verwendet werden, in denen wenige (d.h. bis zu 1, 2 oder 3) Nukleotide in den oben genannten Sequenzen SEQ ID NO:8 bis 19 deletiert, substituiert oder inseriert sind. Zudem können die Sequenzen der Nukleinsäuremoleküle, solange sie die oben genannten Sequenzen SEQ ID NO:8 bis 19 umfassen, eine Größe von 10-300 bp oder im Fall von Einzelsträngen von 10-300 Basen haben, in dem Nukleotide hinzugefügt sind. So können bis zu 1, 2 oder 3 Nukleotide zu den Sequenzen SEQ ID NO:8 bis 19 am 5'- und/oder 3`-Ende hinzugefügt sein. In einer weiteren Ausführungsform können bis zu 5, 7, 10, 20, 30 40, 50, 60, 70, 80, 90 oder 100 oder mehr Nukleotide zu den Sequenzen SEQ ID NO:8 bis 19 am 5'- und/oder 3'-Ende hinzugefügt sein. Insbesondere sind auch solche Nukleinsäuremoleküle umfasst, die eine Länge von mehr als 18, von mehr als 19 oder von mehr als 20, oder bevorzugt von mehr als 21 Basen haben. Bevorzugt sind solche Nukleinsäuremoleküle, die eine Länge von mehr als 25 Basen haben, wobei alle voran genannten Nukleinsäuremoleküle die Sequenzen SEQ ID NO:8 bis 19 umfassen.

In einer weiteren bevorzugten Ausführungsform haben die Nukleinsäuremoleküle im Sinne der Erfindung eine Länge von mehr als 30, 40, 50 oder mehr Basen. Erfindungsgemäß werden auch Nukleinsäuremoleküle bereitgestellt, die eine Länge im Bereich von 21 bis 10.000 Basen haben. Besonders bevorzugt sind Längen der erfindungsgemäßen Nukleinsäuremoleküle, die im Bereich von 18, 19, 20, 21, 22 23, 25 30, 40 oder 50 bis 100 liegen oder die im Bereich von 18, 19, 20, 21, 22 23, 25 30, 40 oder 50 bis 200 liegen oder die im Bereich von 18, 19, 20, 21, 22 23, 25 30, 40 oder 50 bis 300 Basen liegen, insbesondere im Bereich von 23 bis 100 Basen.

In einer bevorzugten Ausführungsform betrifft die vorliegende Erfindung ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist, wobei das Peptid/Nukleinsäurekonstrukt zur Einbringung in Zellen und/oder zum gezielten Einbringen in spezifische Zellen an Moleküle, wie Zell-penetrierende Peptide und/oder Enzymsubstrate, und/oder an Reagenzien, wie Polyethylenimine, Nanocontainer, Nanopartikel oder Lipide, und/oder an Rezeptor-Liganden-Komplexe kovalent oder nicht-kovalent gebunden sind. Diese Modifikationen sind dem Fachmann bekannt und können eingesetzt werden, um die Peptid/Nukleinsäurekonstrukte gezielt in bestimmte Zellen zu übertragen.

Dem Fachmann sind Verfahren bekannt, wie das Peptid/Nukleinsäurekonstrukt zu deren Einbringung in Zellen und/oder zum Erreichen einer Zellspezifität der Nukleinsäuremoleküle, an Moleküle, wie Zell-penetrierende Peptide und/oder Enzymsubstrate, und/oder an Reagenzien, wie Polyethylenimine, Nanocontainer, Nanopartikel oder Lipide, und/oder an Rezeptor-Liganden-Komplexe kovalent oder nicht-kovalent gebunden werden kann. Die genannten Moleküle können an das Peptid und/oder das Nukleinsäuremolekül der vorliegenden Erfindung gebunden/gekoppelt werden. Dabei können kovalente oder nicht-kovalente Bindungen verwendet werden, die dem Fachmann im Rahmen bekannter Verfahren der Kopplungschemie bekannt sind.

Das erfindungsgemäße Peptid wird zur Verwendung in der Reduktion von Nebenwirkungen in Form von immunstimulatorischen Reaktionen/Effekten, die bei einer Gentherapie auftreten, eingesetzt, wobei das Peptid an ein in der Gentherapie eingesetztes Nukleinsäuremolekül, bevorzugt ein siRNA Molekül, gekoppelt ist. Mit anderen Worten wird das Peptid, das an ein in der Gentherapie eingesetztes Nukleinsäuremolekül gekoppelt ist, als Medikament oder als Arzneimittel (therapeutisch) eingesetzt. Beispielsweise können für therapeutische Anwendungen Zellen mittels der erfindungsgemäßen Nukleinsäuremoleküle direkt abgetötet werden, beispielsweise durch den gezielten Einsatz von entsprechenden siRNA-Molekülen. So können beispielsweise gezielt ganz spezifisch Tumorzellen oder Virus-infizierte Zellen abgetötet werden. Deshalb können die vorgeschlagenen Nukleinsäuremoleküle zur Anwendung bei der Behandlung und/oder Vorbeugung von Tumorerkrankungen oder Virus-ausgelöster Erkrankungen verwendet werden. Virus-ausgelöste Erkrankungen im Sinne der Erfindung umfassen Erkrankungen, die beispielsweise von Herpes-Viren, Papilloma-Viren oder HIV-Viren ausgelöst werden. Somit umfassen die Virus-ausgelösten Erkrankungen Krankheiten wie beispielsweise Hepatitis, Zervixkarzinom oder AIDS.

Ebenso sind Nukleotidmoleküle zur Anwendung bei der Behandlung und/oder Vorbeugung von Tumorerkrankungen beschrieben. Tumorerkrankungen, die mit
dem erfindungsgemäßen Arzneimittel behandelt werden können, umfassen Mammakarzinome, Ovarialkarzinome, Bronchialkarzinome, Kolonkarzinome, Melanome, Blasenkarzinome, Magenkarzinome, Kopf/Halstumore, Gehirntumore, Gebärmutterhalstumore, Prostatakarzinome, Hodenkarzinome, Knochentumore, Nierenkarzinome, Bauchspeicheldrüsentumore, Speiseröhrentumore, maligne Lymphome, Non-Hodgkin-Lymphome, Hodgkin-Lymphome und Schilddrüsenlymphome.

Ebenso sind Nukleotidmoleküle zur Anwendung bei der Behandlung und/oder Vorbeugung von Ebolafieber beschrieben. Ebolafieber ist eine Infektionskrankheit, die durch das Ebolavirus hervorgerufen wird. Ebolavirus ist eine Gattung aus der Familie der Filoviridae. Diese Gattung umfasst fünf Spezies und 14 Subtypen von behüllten Einzel(-)-Strang-RNA-Viren. Das Ebolavirus besitzt eine fadenförmige, manchmal auch bazillusförmige Gestalt.

Ebolaviren sind fähig, sich in fast allen Zellen des Wirtes zu vermehren. Dabei kommt es aufgrund der schnellen Virensynthese zu einem Viruskristall (Crystalloid), der vom Bereich des Zellkerns nach außen dringt und einzelne Viren nach Lyse der Zelle freilässt. Das natürliche Reservoir der Ebolaviren (Hauptwirt, Reservoirwirt) konnte bisher nicht zweifelsfrei gefunden werden. Fledermausarten gelten als mögliche Reservoirwirte sowie verschiedene Arten von Flughunden, die in Afrika weit verbreitet sind und eine Infektion mit Ebola-Viren überleben. Eine Übertragung des Virus vom Reservoirwirt auf den Menschen ist bislang ein eher seltener Vorgang, und der genaue Übertragungsweg ist noch nicht vollends geklärt. Nach Angaben der WHO ist eine Übertragung des Virus auf den Menschen auch durch Körperkontakt mit infizierten kranken oder toten Wildtieren wie Affen, afrikanischen Waldantilopen und Flughunden aufgetreten.

Eine Mensch-zu-Mensch-Übertragung der Ebolaviren erfolgt durch direkten Körperkontakt und bei Kontakt mit Körperausscheidungen infizierter Personen per Kontaktinfektion beziehungsweise Schmierinfektion. Weiterhin ist eine Übertragung per Tröpfcheninfektion (aerogene Transmission), durch Geschlechtsverkehr und nach der Geburt (neonatale Transmission) möglich, wobei diese Übertragungswege zahlenmäßig jedoch bislang nur eine untergeordnete Rolle spielen. Die Inkubationszeit variiert zwischen 2 und 21 Tagen. Aufgrund der hohen Letalität (25-90 %) und Infektionsgefahr wird der Erreger in die höchste Risikogruppe 4 nach der Biostoffverordnung eingeordnet. Nach Ablauf der Inkubationszeit treten Symptome ähnlich wie bei einer beginnenden Grippe auf. Dann folgen hämorrhagisches Fieber (hohes Fieber mit >38,5°C in Verbindung mit Blutungen), Leber- und Nierenfunktionsstörungen mit Ödemen, innere Blutungen, Blutungen ins Gewebe (blaue Flecken), blutiger Stuhl und Urin, Schockzustände und Kreislaufzusammenbrüche, Krämpfe und Lähmungserscheinungen, Übelkeit mit Erbrechen, Durchfall sowie Haut- und Schleimhautblutungen. Die Infektion breitet sich auf den ganzen Organismus aus und zerstört die kapillaren Blutgefäße. Insbesondere führen Blutungen im Magen-Darm-Kanal, in der Milz und in der Lunge zum Tode. Auf zellbiologischer Ebene konnte entschlüsselt werden, wie das Ebolavirus in das Zellinnere eindringt. Das Zaire-Ebolavirus aktiviert über einen bislang unbekannten Membranrezeptor der Rezeptor-Tyrosinkinase-Klasse die sogenannte Phosphoinositid-3-Kinase (PI3K) und bewirkt so seine Internalisierung in die Zelle in Form von Endosomen, Inhibitoren der PI3K und nachgeschalteter Enzyme verhinderten die Infektion in Zellkulturversuchen, was Hoffnung auf zukünftige Behandlungsmöglichkeiten gibt. Weitergehend ist bekannt, dass die Expression des Glykorezeptors "liver and lymph node sinusoidal endothelial cell C-type lectin" (LSECtin) auf myeloischen Zellen dem Ebolavirus seine Bindungskapazität verleiht. Fünf Spezies der Ebolaviren werden unterschieden und jeweils nach den Orten ihres ersten bekannten Auftretens benannt: Zaire-Ebolavirus (6 Subtypen), Sudan-Ebolavirus (SEBOV) (3 Subtypen), Cöte d'Ivoire-Ebolavirus (CIEBOV) (1 Subtyp), Bundibugyo-Ebolavirus (BEBOV) und das Reston-Ebolavirus (4 Subtypen). Die vier erstgenannten Spezies verursachen beim Menschen ein hämorrhagisches Fieber mit einer Letalitätsrate von etwa 50 bis 90%.

Derzeit werden von Tekmira Pharmaceutical Corporation siRNA-Sequenzen, die gegen bestimmte Sequenzen des viralen Genoms von Ebola gerichtet sind, im Einsatz gegen Ebolafieber erprobt. Der Wirkstoff "TKM-Ebola" ist ein anti-Ebola-Virus RNAi Konstrukt, der in Primaten einen 100%igem Schutz gegen Zaire Ebola-Virus zeigte. Es wurde jedoch am 3. Juli 2014 berichtet, dass die klinische Phase (TKM Ebola Phase I Clinical Trial) in gesunden Probanden durch die FDA einstweilig gestoppt wurde, da erhöhte Zytokinwerte festgestellt wurden. Erhöhte Zytokinwerte sind ein Anzeichen einer unspezifischen Immunreaktion. Deshalb betrifft die vorliegende Erfindung in einer bevorzugten Ausführungsform ein Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül gekoppelt ist, wobei die Nukleinsäuremoleküle zur Anwendung bei der Behandlung und/oder Vorbeugung von Ebolafieber geeignet sind. Die Natur des Nukleinsäuremoleküls ist, im Sinne des Vorstehenden, nicht auf ein konkretes Nukleinsäuremolekül beschränkt. Ebenso wenig ist die Zielsequenz des Nukleinsäuremoleküls auf eine bestimmte Sequenz limitiert. Vielmehr kann der Fachmann eine geeignete Sequenz des Ebola-Virus-Genoms auswählen und erfindungsgemäß einsetzen.

Zudem kann das erfindungsgemäße Peptid, zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist, in einer weiteren bevorzugten Ausführungsform gegebenenfalls in Kombination mit einem "pharmakologisch verträglichen Träger" und/oder Lösungsmittel formuliert sein. Beispiele für besonders geeignete pharmakologisch verträgliche Träger sind dem Fachmann bekannt und umfassen gepufferte Kochsalzlösungen, Wasser, Emulsionen wie z.B. Öl/Wasser-Emulsionen, verschiedene Arten von Detergenzien, sterile Lösungen, etc.

Arzneimittel im Sinne der Erfindung, die die oben aufgeführten pharmakologisch verträglichen Träger umfassen, können mittels bekannter konventioneller Methoden formuliert werden. Diese Arzneimittel können einem Individuum in einer geeigneten Dosis verabreicht werden. Die Verabreichung kann oral oder parenteral erfolgen, z.B. intravenös, intraperitoneal, subcutan, intramuskulär, lokal, intranasal, intrabronchial oder intradermal, oder über einen Katheter an einer Stelle in einer Arterie. Die Art der Dosierung wird vom behandelnden Arzt entsprechend den klinischen Faktoren bestimmt. Es ist dem Fachmann bekannt, dass die Art der Dosierung von verschiedenen Faktoren abhängig ist, wie z.B. der Körpergröße bzw. dem Gewicht, der Körperoberfläche, dem Alter, dem Geschlecht oder der allgemeinen Gesundheit des Patienten, aber auch von dem speziell zu verabreichenden Mittel, der Dauer und Art der Verabreichung, und von anderen Medikamenten, die möglicherweise parallel verabreicht werden. Eine typische Dosis kann z.B. in einem Bereich zwischen 0,01 und 10000 µg liegen, wobei Dosen unterhalb oder oberhalb dieses beispielhaften Bereiches, vor allem unter Berücksichtigung der oben erwähnten Faktoren, vorstellbar sind. Im allgemeinen sollte sich bei regelmäßiger Verabreichung der erfindungsgemäßen Arzneimittelformulierung die Dosis in einem Bereich zwischen 10 ng- und 10 mg-Einheiten pro Tag bzw. pro Applikationsintervall befinden. Wird die Zusammensetzung intravenös verabreicht sollte sich die Dosis in einem Bereich zwischen 1 ng- und 0,1 mg-Einheiten pro Kilogramm Körpergewicht pro Minute befinden.

Die Arzneimittel der Erfindung können lokal oder systemisch verabreicht werden. Präparate für eine parenterale Verabreichung umfassen sterile wäßrige oder nicht-wäßrige Lösungen, Suspensionen und Emulsionen. Beispiele für nicht-wäßrige Lösungsmittel sind Propylenglykol, Polyethylenglykol, pflanzliche Öle wie z.B. Olivenöl, und organische Esterverbindungen wie z.B. Ethyloleat, die für Injektionen geeignet sind. Wäßrige Träger umfassen Wasser, alkoholisch-wäßrige Lösungen, Emulsionen, Suspensionen, Salzlösungen und gepufferte Medien. Parenterale Träger umfassen NatriumchloridLösungen, Ringer-Dextrose, Dextrose und Natriumchlorid, Ringer-Laktat und gebundene Öle. Intravenöse Träger umfassen z.B. Flüssigkeits-, Nährstoff- und Elektrolyt-Ergänzungsmittel (wie z.B. solche, die auf Ringer-Dextrose basieren). Die erfindungsgemäßen Arzneimittel können außerdem Konservierungsmittel und andere Zusätze umfassen, wie z.B. antimikrobielle Verbindungen, Antioxidantien, Komplexbildner und inerte Gase. Des Weiteren können, abhängig von der beabsichtigten Verwendung, Verbindungen wie z.B. Interleukine, Wachstumsfaktoren, Differenzierungsfaktoren, Interferone, chemotaktische Proteine oder ein unspezifisches immunmodulatorisches Agens enthalten sein.

Des Weiteren können verschiedene erfindungsgemäße Peptide, zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül, bevorzugt ein siRNA Molekül, gekoppelt ist, auch in Kombination zeitgleich oder zeitlich getrennt verabreicht sowie in gleichen oder unterschiedlichen Konzentrationen eingesetzt werden. Handelt es sich um eine siRNA, können so beispielsweise durch die Verwendung verschiedener erfindungsgemäßer Peptid/Nukleinsäuremolekül-Konstrukte eine Vielzahl von Genen effizient ausgeschaltet werden bzw. mRNAs abgebaut werden.

Ferner sind Verfahren zur Behandlung in der Reduktion von Nebenwirkungen in Form von immunstimulatorischen Reaktionen/Effekten, die bei einer Gentherapie auftreten beschrieben, wobei ein in der Gentherapie eingesetztes Nukleinsäuremolekül an
ein Peptid gekoppelt wird und durch die Kopplung an das Nukleinsäuremolekül die Nebenwirkungen, die durch das Nukleinsäuremolekül hervorgerufen werden in Form von immunstimulatorischen Reaktionen/Effekten, reduziert werden. Bezüglich der bevorzugten Ausführungsformen der Verfahren zur Behandlung gilt das oben im Zusammenhang des Peptids der vorliegenden Erfindung gesagte, mutatis mutandis.

Die erfindungsgemäße Reduktion von Nebenwirkungen in Form von immunstimulatorischen Reaktionen/Effekten, die bei einer Gentherapie auftreten, durch die Kopplung der Nukleinsäuremoleküle an Peptide können ggf. mit weiteren Möglichkeiten der Verminderung immunstimulatorischer Effekte durch siRNA, beispielsweise MeO-Modifikation, kombiniert werden.

Außerdem besteht die Möglichkeit, die genannten immunstimulatorischen Effekte gezielt unterstützend zu nutzen, um beispielsweise Zellen zusätzlich zu dem gewollten Gene-Silencing so zu stressen, dass diese selektiv und sicher geschädigt, bzw. abgetötet werden. Dies ist vor Allem bei Zellarten von Vorteil, die sicher abgetötet werden sollen und von ihrem Genom und ihrer Physiologie her sehr flexibel sind und durch das Silencing eines einzelnen oder weniger Gene nicht ausreichend beeinflusst werden, beispielsweise Virus-infizierte und/oder Tumorzellen. Deshalb können also in einer weiteren Ausführungsform Nukleinsäuremoleküle eingesetzt werden, die zunächst erfindungsgemäß an ein Peptid gekoppelt sind, wodurch die immunstimulatorischen Reaktionen oder Effekte reduziert oder unterdrückt werden, und gezielt in bestimmte Zellen eingebracht werden, in denen das Peptid abgespalten wird. Beispielsweise kann in einer bevorzugten Ausführungsform oben beschriebene Nukleinsäuremoleküle (bevorzugt siRNA-Moleküle) eingesetzt werden, die gezielt auf Virus-infizierte und/oder Tumorzellen abzielen und zusätzlich, durch die Abspaltung eines erfindungsgemäßen Peptids in diesen Zellen, immunstimulatorische Effekte auslösen, um so die eigentliche (spezifische) Wirkung der siRNA gezielt in diesen Zellen zu unterstützen.

Beschrieben ist zudem ein Applikationskit zur Modifikation von Nukleinsäuren, Anwendung und Verabreichung der Peptid-modifizierten Nukleinsäuren, bestehend zumindest aus
- wenigstens einer Ampulle (Ampulle A), welche das Peptid zur Bindung an das Nukleinsäuremolekül enthält, und weiter enthalten kann:
- mindestens eine weitere Ampulle (Ampulle B) mit einem Transfektionssystem, beispielsweise Nanopartikel, Polyethylenimine oder Lipide,
- mindestens eine weitere Ampulle (Ampulle C) welche weitere Bestandteile zur Bindung der Nukleinsäuremoleküle und/oder zur Bindung an ein Transfektionssystem enthält,
- Verdünnungs- und Reaktionspuffer für die Inhalte der Ampullen A, B und C
- eine oder mehrere Sonden bzw. Spritzen mit Kanüle und andere benötigte Materialien zur Injektion der Mischung aus den Ampulleninhalten in das die Zielzellen enthaltende Medium sowie
- eine Vorschrift zur Anwendung und Verabreichung.

Die Erfindung soll nachstehend anhand von in der Zeichnung dargestellten Ausführungsbeispielen näher erläutert werden.

Es zeigt:

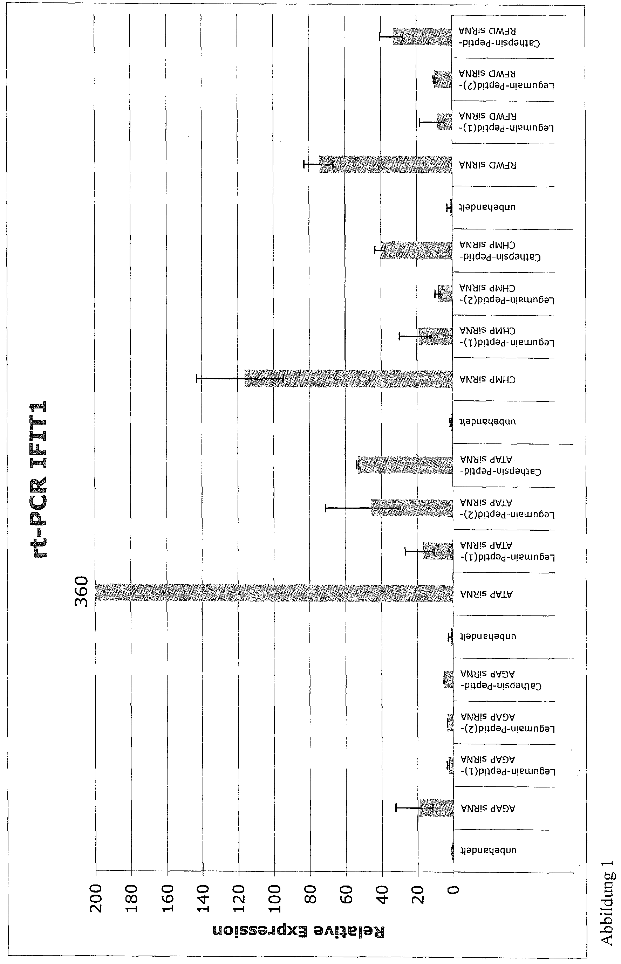

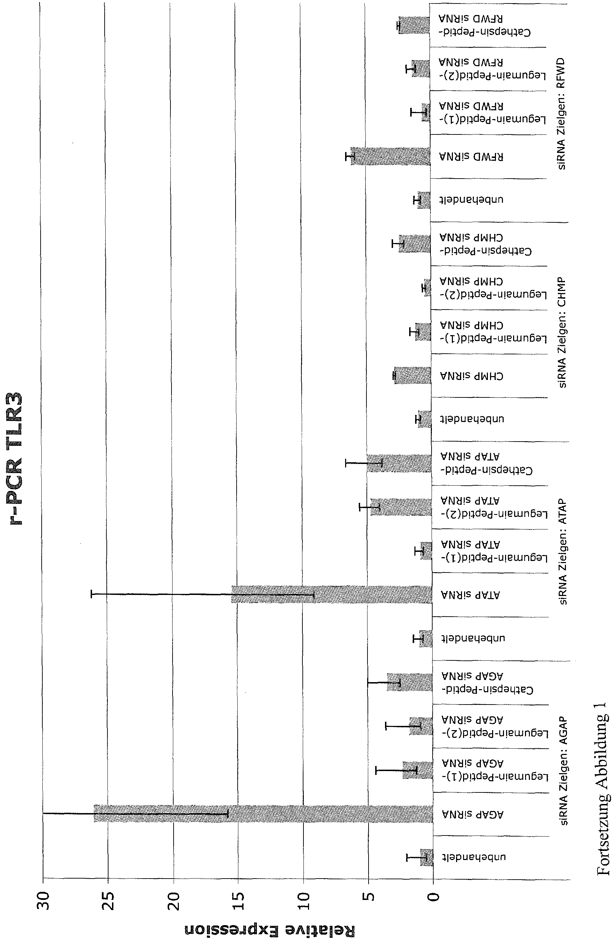

- Abbildung 1:: Darstellung der Expression von IFIT-1 und TLR-3 als Marker für die Induktion immunstimulatorischer Effekte.

Abbildung 1 zeigt die Expression von IFIT-1 und TLR-3 als Marker für die Induktion immunstimulatorischer Effekte, normalisiert auf die Expression in unbehandelten Zellen als Negativkontrolle. In Fig. 1 sind auch die Peptide, die an die siRNA's gekoppelt wurden, angebeben. Während das Transfektionssystem, die Kontroll-siRNA und die freie siRNA jeweils starke immunstimulatorische Effekte induzieren, werden diese durch Peptidbindung jeweils stark vermindert.

### In der Zeichnung bedeuten:

| Transfektionskontrolle: | Leertransfektion ohne siRNA |
|---|---|
| Ctrl-siRNA: | Kontrolltransfektion mit einer Kontroll-(nonsense) siRNA |
| CHMP (1-3): | siRNA gegen CHMP |
| RFWD (1-3): | siRNA gegen RFWD |
| ATAP (1-3): | siRNA gegen ATAP |
| AGAP (1-3): | siRNA gegen AGAP |
| Allstars siRNA Cocktail: | Mischung mehrerer siRNAs für die Induktion von Toxizität. |

Für den Nachweis wurden, wie im folgenden näher beschrieben, die zu behandelnden Zellen in 24-well Zellkulturplatten kultiviert und siRNA-Transfektionen durchgeführt. 24 Stunden nach Transfektion wurde die RNA der Zellen isoliert, in cDNA umgeschrieben und mittels RT-PCR für die jeweiligen Gene analysiert.

Der siRNA-Transfektionsversuch wurde in MCF7-Zellen wie folgt durchgeführt:
Zellkultur: Die adhärent wachsenden Zellen (MCF7, ATCC HTB-22) wurden in Petrischalen in DMEM Medium (10% FCS) unter Standardbedingungen (37°C, 5% CO2, humide Atmosphäre) kultiviert und regelmäßig alle 72 Stunden mittels Trypsinierung behandelt und geteilt. In Vorbereitung auf den Transfektionsversuch wurden jeweils 100.000 Zellen/Well in eine 24-Well-Platte überführt und für 24 Stunden kultiviert.

### Herstellung der siRNA/Peptid-siRNA:

Die verwendeten siRNA-Sequenzen wurden kommerziell synthetisiert und erworben; Firma Axolabs. Im Einzelnen wurden folgende siRNA-Sequenzen synthetisiert:
AGAP (sense; 5'-3') CCC AGA CAA AGA GAA GAA AdTdT
AGAP (antisense; 5'-3') UUU CUU CUC UUU GUC UGG GdTdT
CHMP (sense; 5'-3') AGA UCA UGA UGG AGU UUG AdTdT
CHMP (antisense; 5'-3') UCA AAC UCC AUC AUG AUC UdTdT
AGAP (sense; 5'-3') UGC UCC UGC UCU GAA GAA AdTdT
AGAP (antisense; 5'-3') UUU CUU CAG AGC AGG AGC AdTdT
RFWD (sense; 5'-3') CCA CAG UGC CUC AAU UUG AdTdT
RFWD (antisense; 5'-3') UCA AAU UGA GGC ACU GUG CdTdT

Die Antisense-Stränge wurden jeweils mit einem 5'Aminohexyl-Linker modifiziert; alle siRNAs trugen zwischen den beiden DNA-T-Nukleotiden eine Phosphothioat-Modifikation; wie in Abbildung 1 angegeben, wurden einzelnen Proben an die unten genannten Peptide über den Amino-Linker über eine Peptid-Bindung gekoppelt und mittels RP-HPLC aufgereinigt. Die Reinheit der siRNAs/Peptid-siRNAs lag in allen Fällen über 95% (analysiert durch LC/MS; HPLC; MALDI-TOF). Die siRNAs/Peptid-siRNAs wurden jeweils annealed und entsalzt verwendet. Als Peptide wurden im Einzelnen folgende Sequenzen verwendet:
Legumain-1: Z-Ala-Ala-Asn-Gly-
Lagumain-2: Ac-Gly-Gly-Ala-Phe-Leu-Val-Leu-Pro-Ala-Ala-Asn-Gly-
Cathepsin: Ac-Gly-Gly-Ala-Phe-Leu-Val-Leu-Pro-

Transfektion mit siRNA/Peptid-siRNA: Die jeweiligen siRNA bzw. Peptid-siRNA Sequenzen wurden auf eine Konzentration von 10µmol/l eingestellt und mit Lipofectamine (Firma Invitrogene) komplexiert. Von den Zellen wurde das Medium abgenommen und mit jeweils 2ml/Well PBS gewaschen. Im Anschluss erfolgte die Zugabe von 200µl Optimem-Medium und die Zugabe der Lipofectamine-komplexierten siRNA/Peptid-siRNA in einer finalen Konzentration von 10nmol/l. Nach einer Inkubationszeit von 6 Stunden unter Standardbedingungen wurde das Transfektionsmedium (Optimem mit noch enthaltenen Lipofectamin-siRNA Komplexen) abgenommen und jeweils 1ml Standardmedium (DMEM, 10% FCS) pro Well zugegeben. Das Experiment wurde jeweils in Triplikaten durchgeführt.

Beendigung der Zellkultur, RNA-Isolierung: 24 Stunden nach Zugabe der LipofectaminesiRNA/Peptid-siRNA Komplexe erfolgte die Isolierung der RNA aus den Zellen. Dazu wurde das Kulturmedium aus den Wells entfernt und jedes Well mit jeweils 2ml PBS gewaschen. Im Anschluss wurden jeweils 125µl Trizol-Lösung hinzugegeben und die Zelllysate in Eppendorf-Gefäße überführt. Es folgte die Fällung der RNA mittels Chlorophorm und Ethanol, Zentrifugationsschritte und das Lösen der RNA in Wasser.

cDNA-Synthese und qPCR: Nach Überprüfen der RNA-Qualität mittels MOPS-Gelelektrophorese, wurde die isolierte RNA mittels random-Primer in cDNA umgeschrieben. Im Anschluss erfolgte die Quantifizierung der Genexpression von TLR3 und IFIT1 mittels quantitativer PCR (qTower; Analytik Jena; Cybergreen-System; Primer (5'-3') huIFITl_fwd att tac agc aac cat gag tac aaa; huIFIT1_rev ggc ttc etc att ctg gcc; huTLR3_fwd tca ctt gct cat tct ccc tta c; huTLR3_rev ctg tga gtt ctt gcc caa ttt c). Die PCR-Analysen erfolgten in Triplikaten; die erlangten ct-Werte wurden in das Verhältnis zur Expression von RN18s gestellt, um die relative Expression nach der △△ct-Methode abzubilden.

Die Ergebnisse sind in Abbildung 1 dargestellt.

## Patentansprüche

1. Peptid zur Verwendung in der Reduktion von Nebenwirkungen in Form von immunstimulatorischen Reaktionen/Effekten, die bei einer Gentherapie auftreten, wobei das Peptid an ein in der Gentherapie eingesetztes Nukleinsäuremolekül gekoppelt ist, wobei das Peptid aus bis zu 300 Aminosäuren besteht, wobei das Peptid die Spaltsequenz für Legumain (Spaltsequenz: Ala-Ala-Asn) umfasst.

2. Peptid zur Verwendung nach Anspruch 1, wobei die immunstimulatorischen Reaktionen/Effekte ausgelöst werden durch eine Aktivierung von IFIT1/2, IRF9, TLR3, TLR7, TLR8 oder PKR.

3. Peptid zur Verwendung nach Anspruch 1, wobei die immunstimulatorischen Reaktionen/Effekte zu Rötungen, Entzündungen, grippeähnlichen Symptomen und/oder Fieber führen.

4. Peptid gekoppelt an ein Nukleinsäuremolekül nach einen der Ansprüche 1 bis 3, wobei das Nukleinsäuremolekül weitere chemische Modifikationen umfasst.

5. Peptid zur Verwendung nach einem der Ansprüche 1 bis 4, wobei die Spaltsequenz eine Sequenz mit Hinzufügung von 1 bis 10 Aminosäuren an der Sequenz Ala-Ala-Asn umfasst, und die durch Legumain gespaltet werden kann.

6. Peptid zur Verwendung nach einen der Ansprüche 1 bis 5, wobei das Nukleinsäuremolekül ausgewählt ist aus der Gruppe bestehend aus mRNA, siRNA, miRNA, saRNA, RNA, PNA, DNA, LNA, Gemischen aus verschiedenen Nukleotiden und Aptameren.

7. Peptid zur Verwendung nach einen der Ansprüche 1 bis 6, wobei das Nukleinsäuremolekül eine siRNA ist.

8. Peptid zur Verwendung nach Anspruch 7, wobei die siRNA eine Nucleotidsequenz umfasst, die ausgewählt ist aus der Gruppe bestehend aus:
(5-3) UCA UAU UCG ACU UUG GUU GCC (Polo-like Kinase (PLK); SEQ ID NO:8);
(5-3) UCA AAC UCC AUC AUG AUC U (SEQ ID NO:9) und/oder (5-3) UCC AUC AUG AUC UUC UGG A (SEQ ID NO:10) (CHMP);
(5-3) UUC AUA AAC ACA GUU CUC C (SEQ ID NO:11) (PDCD);
(5-3) UCA AAU UGA GGC ACU GUG C (SEQ ID NO:12) (RFWD);
(5-3) UUU CUU CAG AGC AGG AGC A (SEQ ID NO:13), (5-3) AUA CAC ACC CUU UGC CUC A (SEQ ID NO:14) und/oder (5-3) AUU UCA GGC UCA UAU UCC U (SEQ ID NO:15) (ATAP);
(5-3) CAC AAU UCC CAC UUU GAG C (SEQ ID NO:16), (5-3) GUU ACC CAC AAU UCC CAC U (SEQ ID NO:17) und/oder (5-3) UUU CUU CUC UUU GUC UGG G (SEQ ID NO:18) (AGAP); und
(5-3) UAU UCU CCA AAC AAU GUG C (SEQ ID NO:19) (RCHY).

9. Peptid zur Verwendung nach einem der Ansprüche 1 bis 8, wobei das Peptid/Nukleinsäurekonstrukt zur Einbringung in Zellen und/oder zum gezielten Einbringen in spezifische Zellen an Moleküle, wie Zell-penetrierende Peptide und/oder Enzymsubstrate, und/oder an Reagenzien, wie Polyethylenimine, Nanocontainer, Nanopartikel oder Lipide, und/oder an Rezeptor-Liganden-Komplexe kovalent oder nicht-kovalent gebunden sind.

## Claims

1. A peptide for use in the reduction of side-effects in the form of immunostimulatory reactions/effects which occur with gene therapy, wherein the peptide is coupled to a nucleic acid molecule used in gene therapy, wherein the peptide consists of up to 300 amino acids, wherein the peptide comprises the cleavage sequence for Legumain (cleavage sequence: Ala-Ala-Asn).

2. The peptide for use according to claim 1, wherein the immunostimulatory reactions/effects are induced by activation of IFIT1/2, IRF9, TLR3, TLR7, TLR8 or PKR.

3. The peptide for use according to claim 1, wherein the immunostimulatory reactions/effects lead to redness, inflammation, flu-like symptoms and/or fever.

4. The peptide which is coupled to a nucleic acid molecule according to any one of claims 1 to 3, wherein the nucleic acid molecule comprises further chemical modifications.

5. The peptide for use according to any one of claims 1 to 4, wherein the cleavage sequence comprises a sequence with an addition of 1 to 10 amino acids to the sequence Ala-Ala-Asn, and which can be cleaved by Legumain.

6. The peptide for use according to any one of claims 1 to 5, wherein the nucleic acid molecule is selected from the group consisting of mRNA, siRNA, miRNA, saRNA, RNA, PNA, DNA, LNA, mixtures of different nucleotides and aptamers.

7. The peptide for use according to any one of claims 1 to 6, wherein the nucleic acid molecule is an siRNA.

8. The peptide for use according to claim 7, wherein the siRNA comprises a nucleotide sequence which is selected from the group consisting of:
(5-3) UCA UAU UCG ACU UUG GUU GCC (polo-like kinase (PLK); SEQ ID NO:8);
(5-3) UCA AAC UCC AUC AUG AUC U (SEQ ID NO:9) and/or (5-3) UCC AUC AUG AUC UUC UGG A (SEQ ID NO:10) (CHMP);
(5-3) UUC AUA AAC ACA GUU CUC C (SEQ ID NO:11) (PDCD);
(5-3) UCA AAU UGA GGC ACU GUG C (SEQ ID NO:12) (RFWD);
(5-3) UUU CUU CAG AGC AGG AGC A (SEQ ID NO:13), (5-3) AUA CAC ACC CUU UGG CUC A (SEQ ID NO:14) and/or (5-3) AUU UCA GGC UCA UAU UCC U (SEQ ID NO:15) (ATAP);
(5-3) CAC AAU UCC CAC UUU GAG C (SEQ ID NO:16), (5-3) GUU ACC CAC AAU UCC CAC U (SEQ ID NO:17) and/or (5-3) UUU CUU CUC UUU GUC UGG G (SEQ ID NO:18) (AGAP); and
(5-3) UAU UCU CCA AAC AAU GUG C (SEQ ID NO:19) (RCHY).

9. The peptide for use according to any one of claims 1 to 8, wherein the peptide/nucleic acid construct is covalently or non-covalently linked to molecules such as cell-penetrating peptides and/or enzyme substrates, and/or reagents such as polyethyleneimines, nanocontainers, nanoparticles or lipids, and/or receptor-ligand complexes, for introduction into cells and/or for targeted introduction into specific cells.

## Revendications

1. Peptide pour utilisation dans la réduction des effets secondaires sous la forme d'effets/de réactions d'immunostimulation qui apparaissent lors d'une thérapie génique, le peptide étant couplé à une molécule d'acide nucléique mise en oeuvre dans la thérapie génique, le peptide se composant de jusqu'à 300 acides aminés, le peptide comprenant la séquence de clivage pour la légumaïne (séquence de clivage : Ala-Ala-Asn).

2. Peptide pour utilisation selon la revendication 1, dans lequel les effets/réactions d'immunostimulation sont provoqué(e)s par une activation de IFIT1/2, IRF9, TLR3, TLR7, TLR8 ou PKR.

3. Peptide pour utilisation selon la revendication 1, dans lequel les effets/ réactions d'immunostimulation provoquent des rougeurs, inflammations, symptômes de type grippal, et/ou de la fièvre.

4. Peptide couplé à une molécule d'acide nucléique selon l'une des revendications 1 à 3, la molécule d'acide nucléique comprenant d'autres modifications chimiques.

5. Peptide pour utilisation selon l'une des revendications 1 à 4, dans lequel la séquence de clivage comprend une séquence ayant une addition de 1 à 10 acides aminés à la séquence Ala-Ala-Asn, et pouvant être clivée par la légumaïne.

6. Peptide pour utilisation selon l'une des revendications 1 à 5, dans lequel la molécule d'acide nucléique est choisie dans le groupe constitué d'ARNm, siARN, miARN, saARN, ARN, ANP, ADN, LNA, des mélanges de divers nucléotides et aptamères.

7. Peptide pour utilisation selon l'une des revendications 1 à 6, dans lequel la molécule d'acide nucléique est un siARN.

8. Peptide pour utilisation selon la revendication 7, dans lequel le siARN comprend une séquence nucléotidique choisie dans le groupe constitué de :
(5-3) UCA UAU UCG ACU UUG GUU GCC (kinase de type polo (PLK) ; SEQ ID N° : 8) ;
(5-3) UCA AAC UCC AUC AUG AUC U (SEQ ID N° : 9) et/ou (5-3) UCC AUC AUG AUC UUC UGG A (SEQ ID N° : 10) (CHMP) ;
(5-3) UUC AUA AAC ACA GUU CUC C (SEQ ID N° : 11) (PDCD) ;
(5-3) UCA AAU UGA GGC ACU GUG C (SEQ ID N° : 12) (RFWD) ;
(5-3) UUU CUU CAG AGC AGG AGC A (SEQ ID N° : 13), (5-3) AUA CAC ACC CUU UGC CUC A (SEQ ID N° : 14) et/ou (5-3) AUU UCA GGC UCA UAU UCC U (SEQ ID N° : 15) (ATAP) ;
(5-3) CAC AAU UCC CAC UUU GAG C (SEQ ID N° : 16), (5-3) GUU ACC CAC AAU UCC CAC U (SEQ ID N° : 17) et/ou (5-3) UUU CUU CUC UUU GUC UGG G (SEQ ID N° : 18) (AGAP) ; et
(5-3) UAU UCU CCA AAC AAU GUG C (SEQ ID N° : 19) (RCHY).

9. Peptide pour utilisation selon l'une des revendications 1 à 8, le peptide/la construction d'acide nucléique étant lié(e) de manière covalente ou non covalente à des molécules telles que des peptides pénétrant dans les cellules et/ou des substrats enzymatiques, et/ou à des réactifs tels que les polyéthylène-imines, des nano-récipients, des nanoparticules ou des lipides, et/ou des complexes récepteur-ligands pour l'introduction dans des cellules et/ou pour l'introduction ciblée dans des cellules spécifiques.
